# EUROPÄISCHE PATENTSCHRIFT

(11) **EP 1 091 763 B1**
(45) Veröffentlichungstag und Bekanntmachung des Hinweises auf die Patenterteilung: **17.09.2003**
(21) Anmeldenummer: 99941408.9
(22) Anmeldetag: 29.06.1999
(51) Int. Cl.: A61K 51/10, A61P 31/12

(54) **ANTIVIRALE UND ANTIRETROVIRALE RADIOIMMUNPHARMAKA AUF DER BASIS VON ALPHA- UND BETA-STRAHLERN**
ANTIVIRAL AND ANTIRETROVIRAL RADIOIMMUNOMEDICAMENTS BASED ON ALPHA-EMITTERS AND BETA-EMITTERS
MEDICAMENTS ANTIVIRAUX ET ANTIRETROVIRAUX RADIOIMMUNOLOGIQUES A BASE D'EMETTEURS DE RAYONNEMENT ALPHA ET BETA

(30) Priorität: 29.06.1998 DE 19828732
(43) Veröffentlichungstag der Anmeldung: 18.04.2001
(73) Patentinhaber: Bergter, Wolfgang, 37081 Göttingen (DE)
(72) Erfinder: Bergter, Wolfgang, 37081 Göttingen (DE)
(74) Vertreter: Heitsch, Wolfgang
(86) Internationale Anmeldenummer: DE9901894
(87) Internationale Veröffentlichungsnummer: WO00000223

(56) Entgegenhaltungen:
- EP-A- 0 381 763
- WO-A-91/02541
- WO-A-92/03165
- WO-A-94/07922
- WO-A-99/45969

## Beschreibung

Die vorliegende Erfindung betrifft pharmazeutische Zubereitungen auf der Basis von Radioimmunkonjugaten (RIKen), die mit therapeutischem Ziel in der Virologie, insbesondere zur Bekämpfung bzw. Heilung von durch HIV-1, HIV-2, HIV-3, HTLV-1, HTLV-2, HBV, HCV, HDV, CMV, EBV und HHV8 verursachten Infektionskrankheiten und dadurch induzierten Tumoren, eingesetzt werden sollen. Die vorliegende Erfindung betrifft auch pharmazeutische Zubereitungen auf der Basis von Radioimmunkonjugaten (RIKen), die mit therapeutischem Ziel bei Säugetieren eingesetzt werden sollen, die mit den HIV-1. HIV-2, HIV-3, HTLV-1, HTLV-2, HBV, HCV, HDV, CMV, EBV und HHV8 entsprechenden tierischen Viren (z.B. SIV) infiziert sind.

Die Behandlung der meisten viralen Infektionen - besonders der HIV-, HCV- und HBV-Infektionen - ist bis heute nicht befriedigend gelöst. Nachfolgend soll die Bedeutung dieser Infektionen. der jeweilige Stand der Technik, d.h. die gängigen Therapien, sowie deren Grenzen und Probleme anhand der HIV- und HCV-Infektion exemplarisch dargestellt werden.

### Stand der Technik am Beispiel der HIV-Infektionen

Weltweit waren nach Schätzung der WHO im Dezember 1997 bereits 30,6 Millionen Menschen mit dem humanen Immundefizienzvirus HIV infiziert (http://www.who.org/asd/issues/mar98.htm#th). Die Infektion mit HIV führt zu einem fortschreitenden Immundefekt durch Verlust der mit HIV infizierten CD4-Rezeptor tragenden (CD4+) T-Lymphozyten (Helferzellen). Bei Patienten mit einer symptomatischen HIV-Infektion oder im klinischen Endstadium einer HIV-Infektion (AIDS), bei denen die CD4-Zellzahl unter 350 Zellen/µl Blut liegt und/oder bei denen die Virusload über 30.000 virale RNA Kopien (vRNA)/µl im Serum ist, wird heute eine antiretrovirale Therapie vorzugsweise mit einer oral zu applizierenden medikamentösen Dreierkombination aus zwei Inhibitoren der Reversen Transkriptase (RTI) und einem Protease-Inhibitor (PI) empfohlen (Brodt et al. 1997). Die Replikation der HI-Viren wird zwar durch diese Präparate gestört und verlangsamt, HIV-replizierende Wirtszellen können dadurch jedoch nicht eliminiert werden. Die HIV-Infektion besteht somit als lebensbedrohliche Infektion lebenslang fort. Eine Heilung der HIV-Infektion ist bis heute nicht möglich. Abgesehen davon verschlingt die konventionelle Behandlung eines HIV-Infizierten (AIDS-Patienten) jährlich etwa DM 15,000.-- bei gleichzeitig auftretenden Nebenwirkungen von zum Teil erheblichem Ausmaß.

Konventionelle immuntherapeutische Ansätze zur Behandlung von durch HIV oder HHV8 ausgelösten Erkrankungen (AIDS bzw. Kaposi-Sarkom) stellen bislang keine befriedigende Behandlunesoption dar. HIV und HIV-infizierte Zellen können zwar mit radioaktiv nicht markierten ("kalten") Antikörpern markiert werden, diese lösen aber nicht die gewünschte Immunantwort aus, die zur Elimination der infizierten Zellen führt, weil diese ohnehin durch die HIV-Infektion kompromittiert ist. Die durch die HIV-Infektion gestörte körpereigene cytotoxische Immunantwort muß daher durch einen gezielten, künstlich herbeigeführten Tod möglichst sämtlicher HIV-infizierter Zellen substituiert werden.

### Stand der Technik am Beispiel der HCV-Infektion

Auch eine sehr große Anzahl von Menschen, nämlich über 1 % der Weltbevölkerung, sind mit dem Hepatitis C Virus (HCV) infiziert (Böker und Manns 1997). Die akute HCV-Infektion heilt in 80 % der Fälle nicht spontan. sondem nimmt einen chronischen Verlauf. Ohne Therapie kommt es bei ca. 30 % der Patienten mit chronischer Hepatitis C innerhalb von 20-30 Jahren zur Ausbildung einer Leberzirrhose mit all ihren hepatischen und extrahepatischen Komplikationen: neben portaler Hypertension, Aszitesbildung, Ösophagusvarizenblutung, hepatischer Enzephalopathie und hepatozellulärem Karzinom treten Kryoglobulinämien mit Arthalgien. Pruritus, Purpura, Neuropathie und Glomerulonephritis, kryoglobulinunabhängige Glomerulonephritiden, eine Sicca-Symptomatik, verschiedene Autoimmunerkrankungen und möglicherweise sogar Lymphome auf (Maier 1997, Manns und Rambusch 1997, Petry et al. 1997). Tatsächlich sterben an den Folgen der Hepatitis C-Infektion derzeit weltweit mehr Menschen als an den Folgen der HIV-Epidemie. Die Hepatitis C stellt daher ein riesiges ungelöstes soziales und gesellschaftliches Problem dar.

Als Ursache der bereits seit 1978 als Non-A-Non-B-Hepatitis bekannten Hepatitis C (Alter et al. 1978) konnte 1989 erstmals das Hepatitis C Virus (HCV) identifiziert werden (Choo et al. 1989). Das der Familie der Flaviviren zugeordnete, ca. 30 - 65 nm große Partikel enthält als Genom eine einzelsträngige RNA. die für ein Polyprotein von ca. 3000 Aminosäuren Länge kodiert.

Dieses wird in einem komplexen Vorgang in die drei Strukturproteine C, E1 und E2 sowie die funktionellen Proteine NS1 bis NS5 gespalten und prozessiert (Shimotohno et al. 1995). Die Reifung der Virionen erfolgt im Lumen des endoplasmatischen Retikulums und Golgi-Apparat. Von dort werden sie zur Zelloberfläche transportiert, wo sie aus der Plasmamembran aussprossen (Pozzetto et al. 1996).

Über die Integration viraler oder virusinduzierter Proteine in die Zellmembran HCV-infizierter Zellen ist bislang wenig bekannt (Selby et al. 1993). Es ist aber davon auszugehen, daß Strukturproteine wie E1 und E2, aber möglicherweise auch andere virale und virusinduzierte Proteine an der Oberfläche HCV-infizierter Zellen ausgebildet sind.

Die virale RNA ist durch ein Nukleokapsid aus core-Proteinen (C) geschützt und von einer Lipidhülle mit den darin integrierten Oberflächenproteinen (El und E2) umhüllt (Zilpert und Roggendorf 1997). Mit Hilfe dieser Oberflächenmoleküle erfolgt die Adsorption des Virus, vermutlich über ein von den hypervariablen Regionen teilweise unabhängiges Epitop (Rosa et al. 1996), gezielt an einen Rezeptor der Wirtszelle. möglicherweise an den LDL-Rezeptor (Seipp et al. 1997).

Der Vorgang der Adsorption kann durch Antikörper im Serum HCV-infizierter verhindert werden. Die sogenannten neutralisierenden Epitope unterliegen aber einer lebhaften Mutationstätigkeit des Virus und sind daher derart variabel, daß ein wirksamer Schutz gegen alle Isolate einer Quasispezies nicht aufgebaut werden kann (Farci et al. 1996). Diese ausgeprägte Variabilität erschwert derzeit noch erheblich die Entwicklung einer HCV-Vakzine (Pozzetto et al. 1996).

Auch bei der Behandlung von Hepatitis C-Patienten ist die Medizin noch nicht in der Lage, eine Heilung im engeren Sinn, d.h. ein völliges Eliminieren der die Krankheit auslösenden HC-Viren, zu erzielen. Als einzige Standardtherapie der chronischen Hepatitis C hat sich in den vergangenen Jahren die Behandlung mit Interferon-alpha (IFN-α) etabliert.

IFN-α ist ein körpereigenes Protein, das den Cytokinen zugerechnet wird und von Monozyten und aktivierten B-Lymphozyten als Reaktion auf eine Virusinfektion gebildet wird. Rationale der HCV-Therapie sind die replikationshemmenden und immunmodulatorischen Eigenschaften von IFN-α. Einerseits kann dieses Cytokin den viralen Replikationszyklus hemmen, andererseits kann es cytotoxische T-Zellen aktivieren, die wiederum HCV-infizierte Zellen selektiv abtöten.

Derzeit werden Schemata mit 3 x 6 Mio. Einheiten IFN-α/Woche als subkutane Injektion über 3 - 4 Monate und bei Ansprechen fiir weitere 8 bis 9 Monate empfohlen (Böker und Manns 1997).

Nur ca. 20 % der mit IFN-α behandelten, chronisch HCV-lnfizierten spricht anhaltend auf die Therapie an (Zilbert und Roggendorf 1997). 80 % der Patienten bleiben infiziert und müssen mit den schwerwiegenden Komplikationen der Erkrankung rechnen. Besonders schlecht reagiert der in westlichen Ländern prädominante HCV-Genotyp 1b auf eine IFN-α-Therapie. Der Grund hierfür ist bis heute nicht bekannt.

Die Behandlungskosten für jeden der nach 3 Monaten erfolglos abgebrochenen IFN-α-Therapieversuche (> 50 % der Fälle) belaufen sich derzeit auf ca. 5.000 DM und in allen anderen Fällen auf ca. 20.000 DM, eingeschlossen weitere 25 % Therapieversager, sog. relapser. Eine erfolgreiche IFN-α-Therapie kostet somit mehr als 50.000 DM (umgerechnet nach Berg und Hopf 1997).

Ein breites Spektrum von Nebenwirkungen kann durch IFN-α ausgelöst werden (Berg und Hopf 1997). Fast immer leiden die Patienten wenigstens initial unter zum Teil schweren grippeähnlichen Beschwerden, die einige Patienten sogar zum Therapieabbruch veranlassen. Nicht selten werden auch Blutbildveränderungen, Autoimmunerkrankungen, sowie neurologische und psychische Erkrankungen ausgelöst die häufig von Seiten des Therapeuten einen Behandlunasabbruch erforderlich werden lassen.

Neben der IFN-α-Monotherapie beginnt sich die Kombinationsbehandlung von IFN-α mit Ribavirin zu etablieren. die nach Berichten anderer Arbeitsgruppen (z.B. Schvarcz et al. 1995) und eigenen Erfahrungen (Fetzer et al. 1997) eine höhere Ansprechrate aufweist. Grundsätzliches Problem auch dieser Therapiestrategie bleibt jedoch. daß mit einem Nukleosidanalogon wie Ribavirin nur eine geringe Hemmung der viralen Replikation und keine Elimination des viralen Genoms erzielt werden (Reichard et al. 1993).

Inzwischen zeigte sich, daß eine zunächst erfolgreich therapierte Hepatitis C auch noch nach Jahren wieder aufflammen kann (Vento et al. 1996). Das bedeutet. daß die HCV-Infektion ungeachtet des Einsatzes von IFN-α und Kombinationstherapie mit Ribavirin bislang in den meisten Fällen unheilbar ist.

Ein wirksamer Impfstoff ist auf absehbare Zeit nicht in Sicht. HCV ist wie das humane Immundefizienzvirus HIV durch eine starke Mutagenität im Bereich seiner Oberflächenproteine E1 und E2 gekennzeichnet, besonders im Bereich der neutralisierenden Epitope. Dies ermöglicht es dem Virus als Quasispezies immer wieder, den Angriffen des Immunsystems zu entkommen. Die spontane oder therapeutisch erzielte Elimination eines HCV-Isolats geht auch nicht mit einem wirksamen immunologischen Schutz gegenüber einer Infektion mit einem anderen HCV-Isolat einher (Purcell 1997).

Die klinische Prüfung einer potentiellen Vakzine ist zudem schwierig und zeitaufwendig. Zum einen steht mit der HCV-Infektion von Schimpansen nur ein einziges, schwierig handhabbares Tiermodel zur Verfügung. Zum anderen ist die Prüfung am Menschen nicht risikolos und beansprucht zudem noch Jahre, bevor zuverlässige Aussagen zur Effizienz der Vakzinierung getroffen werden können. Schließlich würde selbst ein bereits heute in den Markt eingeführter HCV-Impfstoff nichts an der Tatsache ändern, daß schätzungsweise 40 Millionen Menschen weltweit HCV-infiziert blieben und einer effektiven antiviralen Therapie bedürften.

Da molekularbiologische Methoden zur gezielten Elimination des HIV- bzw. HCV-Genoms in infizierten Zellen in absehbarer Zeit nicht zur Verfügung stehen werden, muß die Therapie der HIV- und HCV-Infektion die Eliminierung der HIV- bzw. HCV-replizierenden Zellen mit zum gegenwärtigen Zeitpunkt praktikablen therapeutischen Methoden zum Ziel haben.

### Hintergrund der Radioimmuntherapie bei Virus-Infektionen

Den genannten Infektionen ist gemeinsam, daß sie chronisch verlaufen und mit einer hohen Morbidität und Mortalität verknüpft sind. Die Virusreplikation läßt sich inzwischen mit antiviralen bzw. antiretroviralen Pharmaka mehr oder weniger gut supprimieren. Eine Heilung wird derzeit aber bei der Hepatitis B und C nur in einem begrenzten Teil der Fälle erreicht, und eine HIV-Infektion kann durch Hemmung der Virusreplikation allein vermutlich überhaupt nicht eliminiert werden.

Ziel einer effektiven antiviralen bzw. antiretroviralen Therapie muß daher die infizierte Zelle selbst sein.

Die Behandlung mit Radioisotopen ist als Radioiodtherapie aus der Behandlung benigner und maligner Schilddrüsenerkrankungen in Deutschland seit über 50 Jahren als bedeutendstes Verfahren der nuklearmedizinischen Therapie bekannt und hat sich als nebenwirkungsarm erwiesen. Spätkomplikationen. insbesondere eine Malignominduktion. konnten bis heute nicht nachgewiesen werden (Moser 1996).

Neuere Therapiemöglichkkeiten mit Radioisotopen ergeben sich inzwischen mit ¹³¹I-markiertem Meta-Iod-Benzyl-Guanidin (MIBG) bei der Behandlung metastasierter Phäochromozytome und Neuroblastome. durch die Radiosynoviorthese bei der rheumatischen Arthritis, durch intrakavitäre Instillation von ⁹⁰Y-Silikat bei Pleura- oder Peritonealkarzinose. der palliativen Schmerztherapie von Skelettmetastasen mit knochenaffinen Substanzen wie ⁸⁹Sr, oder der Radiophosphorbehandlung der Polycythaemia vera (Moser 1996). In der klinischen Prüfung befindet sich derzeit eine Therapie mit ¹³¹I-markiertem anti-CEA IgG beim kolorektalen Karzinom (Blumenthal et al. 1992, Blumenthal 1994), beim B-Zell-Lymphom (Kaminski et al 1993, Press et al. 1993. Press et al. 1995. Press et al. 1995).

Für die Therapie der HIV-Infektion liegen keine Erfahrungen mit dem Einsatz von Radioisotopen vor. Während Gamma-Strahlung in geringer Dosis offenbar eine verstärkte HIV-Expression bewirken kann (Xu et al. 1996), zeigte sich tierexperimentell anhand der SIV-Infektion eines Makaken-Affen, daß es bei gezielter Lymphknotenbestrahlung zu einer Verminderung der Viruslast im peripheren Blut und einem Stillstand der Krankheitsprogression kommen kann (Fultz et al. 1995). Eine Ganzkörperbestrahlung HIV-infizierter Patienten ist jedoch nur unspezifisch wirksam und erfordert eine hohe Strahlendosis. Der Effekt ist daher prinzipiell unsicher, und der Preis durch unerwünschte Strahlenschäden ist hoch.

Eine solche gezielte Elimination virusinfizierter Zellen ist erfindungsgemäß mit Radioimmunpharmaka möglich, die als radioaktive Komponente einen α- oder β-Strahler enthalten. Die Reichweite der β-Strahlung liegt im Bereich von mehreren Millimetern. Das Problem besteht somit darin. daß eine Schädigung umliegender, nicht infizierter Zellen nicht auszuschließen ist. Günstiger wäre daher im allgemeinen der Einsatz von α-Strahlen. die einen höheren linearen Energietransfer bewirken und deren Strahlung eine geringere Reichweite aufweisen.

Die beschriebenen Probleme werden erfindungsgemäß dadurch gelöst, daß pharmazeutische Zubereitungen bereitgestellt werden, die immunologisch wirksame Moleküle wie monoklonale Antikörper (mAk) oder zelluläre Rezeptoren für die Virusbindung konjugiert mit einem α- oder β-Strahler enthalten. Ein solches Konstrukt wird nachfolgend als Radioimmunkonjugat (RIK) bezeichnet. Auch kleine Peptide. z.B. mittels Moleküldesign entworfen und konstruiert, sind erfindungsgemäβ geeignet, wenn sie, wie Antikörper, an virale Antigene oder zelluläre Antigene, spezifisch fiir die infizierte Zelle, binden. Je geringer das Molekulargewicht solcher Peptide ist. umso leichter passieren sie die Blut-Hirn-Schranke, so daß auch infizierte Zellen im ZNS sicher erreicht und eliminiert werden.

Ein weiterer Aspekt der vorliegenden Erfindung betrifft die Verwendung solcher RIKe zur Herstellung eines Medikaments für die Behandlung von viralen Infektionen und dadurch induzierten Tumoren. Gemäß einer bevorzugten Ausführungsform ist die zu behandelnde Infektion eine HIV-1-, HIV-2-, HIV-3-, HTLV-1-, HTLV-2-, HBV-, HCV-, HDV-, CMV-, EBV- oder HHVB-Infektion bzw. ein durch die Infektion induzierter Tumor beim Menschen bzw. die entsprechende Erkrankung (Infektion mit einem Virus, das den vorgenannten entspricht. und dadurch induzierter Tumor) bei einem Säugetier wie Affe oder Maus.

Ein weiterer Aspekt der vorliegenden Erfindung betrifft einen Kit enthaltend ein erfmdungsgemäßes RIK. vorzugsweise in lyophilisierter Form, eine Säule für chromatographische Trennverfahren sowie ggf. ein Lösungsmittel für den Antikörper/Rezeptor bzw. deren Fragment, ggf. ein Oxidationsmittel wie Chloramin T bzw. einen Chelatbildner zur Durchführung der Konjugation des Radionuklids an das die infizierte Zelle spezifisch erkennende Molekül.

Als RIKe für die erfindungsgemäße pharmazeutische Zubereitung können Verbindungen folgender allgemeiner Formel eingesetzt werden:
a) Bindungsmolekül - Alpha-Strahler;
b) Bindungsmolekül-Beta-Strahler.

Bevorzugt sind insbesondere Verbindungen der Formel
a1) mAk - Alpha-Strahler;
a2) mAk-Fragment - Alpha-Strahler;
a3) Virusrezeptor oder Virusrezeptor-Fragment - Alpha-Strahler;
a4) synthetische Peptide (Moleküldesign) - Alpha-Strahler.

Die Spezifität der mAk soll sich insbesondere gegen Epitope richten, die in die Plasmamembran der Zellen integriert sind. Dazu zählen
a) Epitope der Oberflächen- oder Transmembranglykoproteine des HIV-1. 2 oder 3, z.B. gp 120 und gp41 von HIV-1 und der entsprechenden Strukturproteine von HIV-2, HIV-3:
b) Epitope der Oberflächenglykoproteine des HBV, z.B. HBsAg (SHBs, MHBs, LHBs);
c) Epitope der Oberflächenglykoproteine des HCV, z.B. E1 und E2;
d) Epitope der Oberflächenglykoproteine des HHV8;
e) Epitope der Oberflächen- oder Transmembranglykoproteine anderer Retroviren wie gp46 und gp21 von HTLV-1 1 bzw. HTLV-2;
f) Peptid T20/DP178 (Kilby et al. 1998, Su et al. 1999), oder ein Fragment davon, das einem Abschnitt am C-terminalen Ende von gp 41 entspricht;
g) Epitope der gp220/350-Komplexe von EBV (Darai et al.).

Alternativ dazu können auch (monoklonale) Antikörper Verwendung finden, die spezifisch sind für in die Plasmamembran infizierter Zellen integrierte. virusinduzierte Proteine und Peptide (z.B. des MHC).

Als monoklonale Antikörper kommen in Betracht:
a) murine monoklonale Antikörper;
b) humane bzw. humanisierte monoklonale Antikörper;
c) Antigenbindende Fragmente (Fab. Fab' oder F(ab)₂) muriner oder humaner bzw. humanisierter monoklonaler Antikörper.

Als Virusrezeptoren konnten verschiedene Moleküle identifiziert werden. Derartige Moleküle sollen daher Verwendung finden. unter anderem:
a) CD4-Rezeptoren (bei der HIV-Infektion);
b) LDL-Rezeptoren (bei der HCV-Infektion);
c) ASGPR (Asialoglykoprotein-Rezeptor; Treichel et al 1997) und andere (bei der HBV-Infektion).

Weitere Rezeptoren, wie sie das jeweilige Virus spezifisch zum Eintreten und Infizieren der Zellen benötigt, können verwendet werden in Abhängigkeit der zu therapierenden Infektion. Gegebenenfalls können auch geeignete Antigenbindende Fragmente (Peptide) der Bindungsproteine mit verbesserten pharmakodynamischen und pharmakokinetischen Eigenschaften hergestellt und das jeweilige Bindungsmolekül (oder ein Fragment davon) durch Mutagenese so modifiziert werden. daß verbesserte Bindungseigenschaften für das Radioimmunpharmakon resultieren.

Dementsprechend besonders bevorzugte Verbindungen gemäß o.g. Formeln a) und b) sind
a5) gp41 (von HIV-1) - Alpha-Strahler;
a6) gp120 (von HIV-1) - Alpha-Strahler;
a7) CD4-Rezeptor - Alpha-Strahler;
a8) E l (von HCV) - Alpha-Strahler;
a9) E2 (von HCV) - Alpha-Strahler;
al 0) kleines Peptid gemäß Moleküldesign - Alpha-Strahler.

Geeignete Radionuklide sind ¹³¹I, ³²P, ⁹⁰Y, ⁸⁹Sr, ¹⁷⁷Lu, ¹⁸⁶Re, ¹⁸⁸Re,¹⁰⁵Rh, ⁹⁷Ru, ⁴⁷Sc, ¹⁵³Sm und ¹⁴⁹Tb (Beta-Strahler) bzw. ²¹²Bi, ²²⁵Ac, ²¹³Bi und ²²³Ra (Alpha-Strahler).

Eine therapeutische Option (bei der HIV-Infektion) besteht nun in der Kopplung eines Radioisotops an einen HIV-spezifischen Antikörper, der ein Epitop auf den an der Zelloberfläche HIV-infizierter Zellen präsentierten viralen Strukturproteinen erkennt und daran bindet. Vorzugsweise kommt die Verbindung eines humanen gp41-spezifischen monoklonalen Antikörpers mit einem der folgenden Isotope in Betracht: ¹³¹I, ³²p, ⁹⁰Y, ⁸⁹Sr, ¹⁷⁷Lu, ¹⁸⁶Re, ¹⁸⁸Re, ¹⁰⁵Rh, ⁹⁷Ru, ⁴⁷Sc, ¹⁵³Sm und ¹⁴⁹Tb (Beta-Strahler) bzw. ²¹²Bi, ²²⁵Ac, ²¹³Bi und ²²³Ra (Alpha-Strahler).

Das retrovirale Transmembranglykoprotein gp41 ist in die virale und zelluläre Lipidmembran fest integriert und kann deshalb anders als das Oberflächenprotein gp120 nicht durch shedding vom Virus bzw. von der HIV-infizierten Zelle abdissoziieren. Besonders vorteilhaft sind solche Antikörper, die spezifisch für ein stark konserviertes Epitop sind, wobei humane Antikörper bevorzugt sind.

Gut geeignet als Komponente eines Radioimmunkonjugats und umfassend charakterisiert wäre beispielsweise der humane monoklonale Antikörper *2F5*, der an ein stark konserviertes und daher bei den unterschiedlichen HIV-1-Isolaten weit verbreitetes Epitop bindet (Muster et al. 1993).

Eine andere Option (bei HIV-Infektion) besteht in einer entsprechenden Konjugation von CD4-Molekülen bzw. Fragmenten von CD4-Molekülen mit Radioisotopen. Sowohl α- als auch β-Strahler wie ¹³¹I, ³²P, ⁹⁰Y,⁸⁹Sr, ¹⁷⁷Lu, ¹⁸⁶Re, ¹⁸⁸Re, ¹⁰⁵Rh, ⁹⁷Ru ⁴⁷Sc, ¹⁵³Sm und ¹⁴⁹Tb (Beta-Strahler) bzw. ²¹²Bi, ²²⁵Ac, ²¹³Bi und ²²³Ra (Alpha-Strahler) können Verwendung finden. Die Konjugate können wirksam verwendet werden, um eine Heilung der Infektion mit HIV-1 oder HIV-2 durch gezielte Abtötung aller infizierter Zellen zu erzielen.

CD4-Moleküle sind in die Zellmembran von T-Lymphozyten integriert, haben ein Molekulargewicht von etwa 55.000 Dalton und spielen mit ihrem nach außen gerichteten Rezeptoranteil eine wichtige Rolle bei der Infektabwehr (Gaubin et al 1996), aber auch für die Bindung von HIV an diese Zellen (Dalgeish et al. 1984). HI-Viren bedienen sich des CD4-Rezeptors als Ankopplunasort auf der Außenseite der Zellmembran. Die genaue Stelle, an der das virale gp120-Oberflächenglykoprotein bindet. konnte als das aminoterminale Ende des CD4-Moleküls lokalisiert werden. Es wird als V1 -Domäne bezeichnet (Richardson et al. 1988, Arthos et al. 1989).

Aufgrund dieser Kenntnisse sind bereits eine Reihe von Untersuchungen unternommen worden, die klären sollten, ob synthetische CD4-Moleküle oder Teile dieses Proteins (Peptide) therapeutisch einsetzbar sind. Es ließ sich zeigen, daß derartige Moleküle die CD4-Bindungsstelle des viralen Oberflächenglykoproteins gp120 belegen und HI-Viren neutralisieren (Deen et al. 1988, Bym et al. 1989. Clapham et al. 1989, Watanabe et al 1989). Auf diese Weise können uninfizierte Zellen vor einer Infektion mit HIV geschützt werden. Für die Therapie einer bestehenden HIV-Infektion reicht die Applikation solcher Proteine oder Peptide allein jedoch nicht aus. weil das HIV-Genom in den infizierten Zellen dadurch nicht zerstört wird und neue HI-Viren ungehindert weiter repliziert werden.

Eine weitere Option (bei HIV- und HCV-Infektion) sind Konjugate aus einem monoklonalen Antikörper oder dessen antigenbindendem Fragment bzw. dem Rezeptormolekül des HIV bzw. HCV oder einem Fragment dessen und einem α- oder β-Strahler.

Die Herstellung muriner. humanisierter und humaner monoklonaler Antikörper ist bekannt (Übersichten: Lidell und Weeks 1995, Peters et al. 1996). Die Isolierung SIV- und HIVspezifischer monoklonaler Antikörper ist eine Grundlage der HIV-Forschung (Bergter 1990). Die Gewinnung und Konjugation radioaktiver Isotope ist ebenfalls hinreichend beschrieben (Übersicht in: Eckert und Kartenbeck 1996).

Alpha-Strahlung ist eine Korpuskularstrahlung. Durch Emission eines Alpha-Teilchens verliert der Kern eines Alpha-Strahlers eine positive Ladung von +2*e* und eine Masse von etwa 4 amu. Die emittierten Alpha-Teilchen besitzen eine mit weniger als 100 µm deutlich geringere Reichweite als Elektronen von Beta-Strahlern. Sie weisen aber eine höhere Energie auf und bewirken eine wesentlich dichtere Ionisation als Beta-Strahler (Zalutzky und Bigner 1996). Dies führt zu einer intensiveren Schädigung der Plasmamembranen infizierter Zellen und einer deutlich gesteigerten Cytotoxizität. während nichtinfizierte Zellen einer geringeren unspezifischen Strahlenwirkung ausgesetzt sind. Dies ist wichtig, wenn sich beispielsweise HIV-infizierte Zellen disseminiert im gesamten Körper des Patienten verteilen.

Von den über 100 Radionukliden, die Alpha-Teilchen emittieren, eignen sich die meisten wegen ihrer langen Halbwertszeit nicht für eine Radioimmuntherapie. Viele können zudem nur schwer und in zu geringer Quantität hergestellt werden, so daß bislang nur wenige Alpha-Strahler für die klinische Erprobung in Betracht gezogen worden sind. Die meisten Erfahrungen liegen mit der Anwendung von Astat-21 1 (²¹¹At) und Wismut-212 (²¹²Bi) vor (Zweit 1996).

Astat-211 ist für die erfindungsgemäßen therapeutischen Zwecke gut geeignet. weil es auf zwei Wegen zerfällt, die beide zur Emission von Alpha-Teilchen führen, weil es Röntgenstrahlung aussendet. die eine ausreichende Intensität aufweist, um Blut- oder Gewebeproben mit einem Gamma-Zähler zu untersuchen, oder um das therapeutische Verfahren mit bildgebenden Verfahren wie der Single-Photon-Emissions-Computertomographie (SPECT) zu analysieren (Turkington et al. 1993), und weil es eine Halbwertzeit (HWZ) von nur 7 h hat.

Ebenfalls vorteilhaft im Sinn der vorliegenden Erfindung ist Wismut-212 (HWZ: 70 min), das bereits mit monoklonalen Antikörpern konjugiert und im Tiermodell erfolgreich zur Elimination maligner Zellen eingesetzt worden ist (Kozak et a. 1986, Macklis et al. 1988).

Ein anderer geeigneter Alpha-Strahler, mit einer Halbwertszeit von 46 Minuten, ist Wismut-213 (²¹³Bi), das mittels eines Generators aus dem Mutternuklid Actinium (²²⁵Ac) erzeugt werden kann.

Allgemein ausgedrückt sind alle solche Alpha- und Beta-Strahler besonders geeignet, die eine HWZ von 10 d oder darunter haben, da die Patienten dann nur über einen relativ kurzen Zeitraum einer nennenswerten Dosis Radioaktivität ausgesetzt werden.

Die Wirkungen radioaktiver Strahlung auf Zellen und genetisches Material (DNA) sind hinreichend belegt (Übersichten in: Kauffmann et al. 1996, Sauer 1998). Die Grundlage der strahlenbiologischen Wirkungen stellt die strukturelle Schädigung der genetischen Information (DNA) virusinfizierter Zellen dar (im Falle der HCV-Infektion die hepatozelluläre DNA). Derart veränderte zelluläre DNA kodiert fehlerhafte Proteine, die ihre Funktion einbüßen. So verlieren nicht nur Enzyme. die für die Virus-Replikation unentbehrlich sind, ihre Funktion, sondern es kommt darüber hinaus auch zu Stoffwechselveränderungen in der virusinfizierten Zelle. Dies führt zur Eliminierung der virusinfizierten Zellen durch reproduktiven Zelltod oder Apoptose. Darüber hinaus können Viren durch ionisierende Strahlung auch direkt inaktiviert werden (Follea et al. 1996).

Unter onkologischen Gesichtspunkten wird Bestrahlung erfolgreich zur Therapie des AIDSassoziierten Kaposi-Sarkoms eingesetzt, das durch das Humane Herpes-Virus 8 (HHV 8) induziert wird (Conill et al. 1997).

Erfahrungen zur Wirkung von ionisierender Strahlung auf die HIV-Replikation liegen kaum vor. Während Gamma-Strahlung in geringer Dosis offenbar eine verstärkte HIV-Expression bewirken kann (Faure et al. 1995, Xu et al. 1996), fiel bislang keine Akzeleration der HIV-Replikation infolge therapeutischer Bestrahlung HIV-assoziierter Tumore auf (Lotz et al. 1990. Plettenberg et al. 1991. Scheidegger et al. 1991, Stanley et al. 1991, Krain und Dieckmann 1994. Saran et al. 1995, Swift 1996, Saran et al. 1997). Tierexperimentell zeigte sich vielmehr anhand der SIV-Infektion eines Makaken-Affen. daß es bei gezielter Lymphknotenbestrahlung zu einer Verminderung der Viruslast im peripheren Blut und einem Stillstand der Krankheitsprogression kommen kann (Fultz et al. 1995). Eine Ganzkörperbestrahlung HIV-infizierter Patienten ist jedoch nur unspezifisch wirksam und erfordert eine hohe Strahlendosis. Der Effekt ist daher prinzipiell unsicher und der Preis durch unerwünschte Strahlenschäden hoch.

Der gezielte Einsatz von freien Radioisotopen zur Behandlung von viralen (HIV-1, HTLV-1, HBV, HHV8, HIV-2, HIV-3, HTLV-2, HCV, HDV, CMV, EBV, etc.) Infektionen ist hingegen bislang nicht erprobt worden. Er findet seine Legiti-mation aber in der Therapie solcher Infektionskrankheiten, die durch ihren chronischen Verlauf, die Häufigkeit und Schwere ihrer Folgeerkrankungen und eine mangelhafte konventionelle Therapierbarkeit charakterisiert sind.

Grundlage einer Radioimmuntherapie der oben genannten viralen Infektionen mit den erfindungsgemäßen Immunglobulin-Konjugaten ist, daß virale Antigene oder Virusinduzierte Antigene in die äußere Membran infizierter Zellen integriert werden. Dies ist eine Eigenschaft, die für viele Viren bereits belegt wurde, so fiir die Flaviviren (Westaway und Goodman 1987, Ng et al. 1992), HSV (Schlehofer et al. 1979), Influenza-Viren (Boulan et al. 1980. Ciampor et al. 1981. Kohama et al. 1981. Hughey et al. 1992), Rabies-Viren (Revilla-Monsalve et al. 1985), EBV (Liebowilz et al. 1986). HBV (Saito et al. 1992. Gerber et al. 1988. Chu et al. 1997) und HIV (Timar et al. 1986. Rusche et al. 1987. Feremans et al. 1988, Desportes et al. 1989. Ikuta et al. 1989. Dennin et al. 1991. Dudhane et al. 1996).

Für die erfindungsgemäßen Radioimmunpharmaka bzw. RIKe auf Basis von Immunglobulinen sollen daher als immunologisch wirksame Komponenten der Radioimmunkonjugate monoklonale Antikörper oder deren Antigenbindende Fragmente. die jeweils ein Epitop der viralen bzw. virusinduzierten Antigene auf der Plasmamembran infizierter Zellen erkennen und binden, Einsatz finden. Besonders geeignet sind monoklonale Antikörper gegen ein möglichst stark konserviertes Epitop eines viralen Antigens. Der an den monoklonalen Antikörper bzw. dessen Antigenbindendes Fragment konjugierte Alpha- bzw. Beta-Strahler schädigt dann gezielt die Virusreplizierende Zelle. Im Fall der Behandlung der HCV-Infektion sind Antikörper gegen ein möglichst stark konserviertes Epitop auf den Proteinen E1 oder E2, oder gegen das von der NS2-Region kodierte Transmembranprotein des HCV (Santolini et al. 1995) bevorzugt.

Eine besonders bevorzugte Ausführungsform der vorliegenden Erfindung betrifft die gleichzeitige Applikation von "kalten", d.h. nichtradioaktiv markierten monoklonalen Antikörpern und Rezeptoren bzw. deren Fragmenten. Die Verabreichung der kalten Antikörper/Rezeptoren/Fragmente kann vor (pretargeting), nach oder gleichzeitig mit den Radioimmunkonjugaten gemäß der vorliegenden Erfindung erfolgen, wodurch freie Viruspartikel abgefangen und neutralisiert werden können. Der kalte Antikörper kann identisch sein mit dem "heißen", er kann aber auch ein anderer sein. Wesentlich ist lediglich, daß er an die infizierte Zelle bindet.

Hintergrund einer Radioimmuntherapie viraler Infektionen mit Zellrezeptor-Konjugaten ist neben der Expression viraler Proteine auf der Oberfläche infizierter Zellen auch, daß die Adsorption der Viren an die Wirtszellen gezielt über einen Rezeptor auf der Zellmembran vermittelt wird. Eine derartige Rezeptorvermittelte Adsorption wurde bereits für zahlreiche Viren belegt, beispielsweise für HIV (Dalgleish et al. 1984), EBV (Fingeroth et al. 1984), Polioviren (Leon-Monzon et al. 1995), Rabiesviren (Lentz et al. 1986), Influenza-C-Viren (Nishimura et al. 1988), Masern-Viren (Dorig et al. 1993) HAV (Kaplan et al. 1996), HSV (Whitbeck et al. 1997), HBV (Treichel et al. 1997), HCV (Seipp et al. 1997). Coxsackie B3 (Shafren et al. 1997) und Adenoviren (Bergelson et al. 1998), um nur einige zu nennen. Somit lassen sich mittels z.B. Radioimmunkonjugat a7) HIV-Infizierte behandeln. Genauso kann die Behandlung von Hepatitis C-Patienten mit einem Konjugat aus LDL-Rezeptor (oder einem Fragment dessen) sowie Radionuklid, vorzugsweise α-Strahler, besonders bevorzugt Wismut-213 oder Astat-211, erfolgen, da das HCV über den LDL-Rezeptor als zellulären Rezeptor (Seipp et al. 1997) in die Zellen gelangen könnte.

Entsprechende rekombinante Rezeptormoleküle binden an die auf der Oberfläche infizierter Zellen exprimierten viralen Antigene und koppeln in der oben geschilderten Weise Radioisotope an die infizierte Zelle.

Die Erfolge der erfindungsgemäßen Therapie mit den oben beschriebenen Radioimmunkonjugaten sind bei vorher- oder einhergehender Verminderung der Viruslast durch Behandlung des Patienten sogar noch zu verbessern. Diesbezüglich geeignet sind die moderne antiretrovirale Standardtripeltherapie bei HIV-Infektionen und die IFN-α-Mono- oder Kombinationstherapie mit Ribavirin bei HBV- und HCV-Infektionen. Durch die Tripeltherapie kann eine höhere Dosis des Anti-HIV-Radioimmunpharmakons an die virusinfizierten Zellen herangebracht werden, um diese zu eliminieren. Andernfalls würde das RIK von freien Viruspartikeln abgefangen und die cytotoxische Wirkung vermindert werden. Die IFN-α-Monooder Kombinationstherapie mit Ribavirin verursacht die Stabilisierung der Hepatocyten-Zellmembran, was die Viruspartikel am Aussprossen hindert.

Dementsprechend bevorzugt ist ein pharmazeutisches Kombinationprodukt, das neben den RIKen auch IFN-α und/oder Ribavirin und/oder einen Protease-Inhibitor (PI) und/oder ein antiretrovirales Nukleosid-Analogon wie AZT, das in Standardtherapien bei HIV-Infektionen verwendet wird, enthält. Ebenfalls bevorzugt sind solche Produkte, die zusätzlich unmarkierte Antikörper. Rezeptoren oder deren Fragmente gemäß der Definition in Patentanspruch 1 a) bis c) enthalten.

Eine weitere bevorzugte Variante für die Behandlung der infizierten Patienten mit den erfindungsgemäßen RIKen besteht darin, (noch) nicht infizierte Zellen vor Virusbefall dadurch zu schützen, daß die entsprechenden Rezeptoren (im Fall von HIV den CD4-Rezeptor) mit unmarkierten Molekülen blockiert werden. Besonders geeignet dafür sind anti-CD4-Rezeptor-Antikörper und solche Fragmente der Antikörper, die eine ausreichend große Affinität für den Rezeptor haben. Auch synthetische Peptide mit Rezeptor-Affinität sind geeignet und können vor gleichzeitig mit oder auch nach der Verabreichung der RIKe appliziert werden. Eine vor- und gleichzeitige Gabe ist bevorzugt.

Das erfindungsgemäße Radioimmunkonjugat wird parenteral, vorzugsweise intravenös, appliziert. Dazu ist im allgemeinen eine mehrtägige (2 bis 10 Tage, im allgemeinen 3 bis 7 oder 8 Tage) stationäre Unterbringung erforderlich, um den Patienten bis zum Abklingen der Strahlung von der Umgebung abzuschirmen.

Das RIK bindet spezifisch (mittels monoklonaler Antikörper, deren Fragmenten bzw. mittels Rezeptoren und deren Fragmenten) an das entsprechende Epitop eines in die Zellwand der Virusreplizierenden Zelle integrierten retroviralen Transmembran- oder Oberflächenslykoproteins (oder des Rezeptors). Das somit an dieser Zelle fixierte Radioisotop gibt Strahlung an die nähere Umgebung ab. Dabei wird insbesondere die mit zahlreichen Radioimmunkonjugaten besetzte virusinfizierte Zelle geschädigt.

Nach Applikation des Radioimmunpharmakons (auch bei HIV-Patienten) muß dennoch vorübergehend besonders mit hämatologischen und hepatischen Nebenwirkungen und einem erhöhten Risiko für opportunistische Infektionen gerechnet werden, denn es ist zu erwarten, daß neben einer allgemeinen Myelotoxizität die Zahl der T4-Helferzellen erheblich abfallen wird. Da es sich hierbei aber um den Verlust von in ihrer normalen Funktion gestörten HIV-infizierten T4-Helferzellen handelt. ist dies ein erklärtes Therapieziel. Gegebenenfalls ist aus diesem Grund die Radioimmuntherapie mit einer Stammzelltransplantation zu verbinden. Da T4-Vorläuferzellen jedoch wegen fehlender CD4-Rezeptoren nicht mit HIV infiziert sind, wird eine Regeneration der T4-Helferzellpopulation erfolgen. Dieses Phänomen wird auch in der vorbeschriebenen tierexperimentellen Untersuchung beobachtet (Fultz et al. 1995).

Eine bevorzugte Ausführungsform der Erfindung bezieht sich auf das Konjugat a7) und dessen Verwendung zur Behandlung von HIV-Infektionen. CD4-Moleküle können in großem Maßstab aus Gewebekulturen gewonnen werden (Deen et al. 1988. Glick und Pasternak 1995). Die Reinigung der CD4-Moleküle ist mit Hilfe etablierter molekularbiologischer Reinigungsverfahren möglich. Sie können beispielsweise direkt aus der Zellmembran durch differentielle Extraktion mit Hilfe nichtionischer Detergentien isoliert werden (Eckert und Kartenbeck 1997 ^{l}). durch Reinigung aus Gewebekulturüberständen wie bei Deen et al. 1988 beschrieben, durch eine biomagnetische Separation (Deutsche Dynal GmbH, Hamburg) oder andere Methoden. Die Herstellung von Konjugaten aus Proteinen und radioaktiven Isotopen kann beispielsweise mit der Chloramin T-Methode erfolgen (Hunter und Greenwood 1962, Eckert und Kartenbeck 1997 ²).

Eine andere Ausgestaltung dieser Ausführungsform betrifft die Verwendung von synthetischen CD4-Fragmenten mit gp120-Affinität. Solche Fragmente lassen sich in einem prokaryotischen Wirt wie *Ezcherichia coli* oder einem eukaryotischen Wirt wie *Saccharomyces cerevisae* in großen Mengen produzieren (Wilcox und Studnicka 1988. Martin und Scheinbach 1989).

Schließlich können die CD4-Fragmente durch Mutagenese (Jones et al. 1990) zusätzlich modifiziert und für die therapeutische Anwendung bezüglich ihrer Pharmakokinetik, gp120-Affinität und Liquorgängigkeit optimiert werden. Voraussetzung für die gezielte Mutagenese sind Kenntnisse über die Rolle der Aminosäuren im funktionstüchtigen Peptid. Diese lassen sich durch genetische Analysen. Röntgenstrukturanalyse der dreidimensionalen Peptidstruktur gewinnen und mit Hilfe von rechnergestützten Untersuchungen am Computer simulieren und analysieren. Ein besonders bevorzugtes Konjugat a7) besteht aus CD4-Rezeptor und einem der Radionuklide Bi-212. Bi-213 oder At-211.

Der bedeutendste Vorteil einer Therapie mit den erfindungsgemäßen Radioimmunkonjugaten gegenüber der jeweiligen Standardtherapie (HCV: IFN-α-Therapie; HIV: konventionelle antiretrovirale Therapie) besteht darin, daß virusinfizierte Zellen direkt geschädigt und eliminiert werden, wodurch die Chance auf eine komplette Heilung deutlich steigt. Dies gilt auch für das durch die HCV-Rezidivproblematik belastete Verfahren der Lebertransplantation bei HCVbedingter Leberzirrhose.

Ein Vorteil der erfindungsgemäßen Radioimmuntherapie, insbesondere bei Verwendung des Konjugats a7), ist, daß die virusinfizierten Zellen selektiv geschädigt werden. Die Radioimmunkonjugate binden spezifisch mit Hilfe des Protein- bzw. Peptidanteils an das (im Fall der HIV-Infektion) CD4-spezifische Epitop retroviraler gp 120-Oberflächenglykoproteine, die auf der äußeren Cytoplasmamembran HIV-replizierender Zellen für die Virusknospung exponiert werden und geben ihre Strahlung direkt an die Zelle ab.

Ein weiterer Vorteil dieser Konjugate a7) ist, daß sie für die Behandlung eines breiten Spektrums von Virus-Stämmen (HIV-1. HIV-2. HIV-3. HTLV-1, HTLV-2, etc.) geeignet sind. Dies ist angesichts der Mutationsfreudigkeit dieser Viren und der damit verbundenen Resistenzentwicklungen gegenüber herkömmlichen therapeutischen Substanzen enorm wichtig (die für die CD4-Rezeptorbindung relevanten gp120-Epitope sind nämlich bei allen HIV-1- und HIV-2-lsolaten sehr ähnlich (Sattentau et al. 1988) und für die Infektiosität essentiell.)

Ein weiterer Vorteil synthetischer und gegebenenfalls weiter modifizierter Peptide mit möglichst geringer Größe. aber noch erhaltener Antigen-Spezifität, besteht darin, daß sie die Blut-Hirn-Schranke leicht passieren können. Solche Konjugate sind daher auch zur Elimination infizierter Zellen des ZNS geeignet.

Entscheidend ist die Tatsache. daß ein CD4-Molekül bzw. ein Teil des CD4-Moleküls mit einem Radionuklid gekoppelt wird. um damit die HIV-Infektion mit dem Ziel der Elimination virusinfizierter Zellen zu therapieren. Der Nutzen der beschriebenen nuklearmedizinischen Therapie von HIV-Infektionen ergibt sich allein daraus, daß es sich bei dieser Virusinfektion um eine im Vergleich zu anderen viralen Infektionen gefährliche, chronisch verlaufende und vermutlich in fast allen Fällen zum Tode führende Infektion mit Lentiviren handelt, die mit den üblichen Pharmaka nicht zu heilen ist. Da gegen die HIV-Infektion außerdem auf längere Sicht kein zuverlässiger Impfstoff zur Verfügung stehen wird (Gallo, Reuters News, Mai 1997) und inzwischen gegen herkömmliche antiretrovirale Mittel einfach und mehrfach resistente HIV-Stämme aufgetreten sind (Erickson und Burt 1996. Imrie et al. 1997), gewinnen neuartige Therapiestrategien wie die hier beschriebene große Bedeutung.

Die Verfahren zur Gewinnung von CD4-Molekülen, Entwicklung von CD4-Fragmenten mit gp 120-Affinität durch DNA-Rekombinationstechniken und zur in vitro Markierung löslicher Proteine mit Radioisotopen sind Stand der Technik. Die Konjugation von Radioisotopen an CD4-Moleküle oder entsprechende CD4-Fragmente wird in den Beispielen veranschaulicht.

Schließlich liegt ein großer Vorteil in der zur üblichen antiretroviralen Therapie vergleichsweise guten subjektiven Verträglichkeit und objektiven Nebenwirkungsarmut radioimmunologischer Therapien. Zwar ist bekannt, daß die Strahlungsenergie eines α- oder β-Strahlers wie ¹³¹I eine Reichweite von etwa einem bis zu 40 Zelldurchmessern hat. Maligne Transformationen gesunder Zellen als Folge einer Strahlenschädigung sind aber extrem selten und statistisch kaum zu erfassen. Wird eine gesunde Zelle geschädigt, so verliert sie ebenfalls in den meisten Fällen ihre Teilungsfähigkeit und es tritt der reproduktive oder der programmierte Zelltod (Apoptose) ein. Die Bestrahlung umliegender gesunder Zellen spielt bei der Radioimmuntherapie HIV-infizierter T-Lymphocyten bzw. HCV-infizierter Hepatocyten auch deswegen eine untergeordnete Rolle, weil sich diese Zellen überwiegend in der Zirkulation befinden und gesunde Zellen daher nur kurzzeitig einer Strahlenbelastung ausgesetzt sind.

### Herstellung von Radioimmunkoniugaten

### 1. Radioimmunpharmaka auf der Basis von monoklonalen Antikörpern

Die Entwicklung virusspezifischer monoklonaler Antikörper und die Überprüfung ihrer Kreuzreagibilitäten sind Grundlagen virologischer Forschungsarbeiten (Bergter 1990).

Identifizierung und Reinigung zellmembrangebundener viraler Proteine oder Peptide sind Stand der Technik (Eckert und Kartenbeck 1997). Ebenso sind die Methoden zur Herstellung muriner, humanisierter und humaner monoklonaler Antikörper sowie die Präparation Antigenbindender mAk-Fragmente bekannt (Peters und Baumgarten 1990. Lidell und Weeks 1996). Identifizierung und Isolierung viraler und virusinduzierter Antigene aus der Plasmamembran infizierter Zellen und die Erzeugung monoklonaler Antikörper werden in Beispiel 1 skizziert.

### Beispiel 1:

Durch immuncytologische und elektronenmikroskopische Voruntersuchungen wird zunächst geklärt, ob prinzipiell virale Proteine in die Zellmembran infizierter Zellen integriert sind, die einer Radioimmuntherapie zugänglich wären. Dazu wird die Bindung von virusspezifischen Antikörpern an infizierten Zellen durch Inkubation mit markierten murinen monoklonalen Antikörpern fluoreszenzmikroskopisch oder elektronenmikroskopisch nachgewiesen (Payne et al. 1990, Stirling 1990, Kaito et al. 1994, Sabri et al. 1997).

Lassen sich derartige Antigene in der Cytoplasmamembran nachweisen, muß geklärt werden, um welche Antigene es sich im einzelnen handelt. Hierzu werden virusinfizierte Zellen Ivsiert und die Membranproteine gelelektrophoretisch aufgetrennt. Auf Nitrozellulose geblottet werden sie dann mit Antiseren oder murinen monoklonalen Antikörpern analysiert.

Für die Immunisierung und Gewinnung monoklonaler Antikörper ist die Reinigung der gefundenen. in die Zellmembran integrierten, viralen oder virusinduzierten Antigene erforderlich. Dazu werden Viren in geeigneten Zellkultursystemen, beispielsweise HIV in H9-Zellen, MT-4-Zellen. MOLT-4-Zellen, HUT-78-Zellen u.a. (Bergter 1990) und HCV in DAUDI-Zellen (Nakajima et al. 1996), kultiviert und aus dem Zellkulturüberstand isoliert. Zunächst werden die Zellen und Zelltrümmer abzentrifugiert. Anschließend wird das Virus aus dem gereinigten Überstand durch Zentrifugation bei 27.000 x g sedimentiert. Das Viruspellet wird dann in Probenpuffer resuspendiert und gelelektrophoretisch aufgetrennt. Schließlich wird die Antigenfraktion isoliert, die dem gesuchten Antigen auf der Plasmamembran entspricht. Dieses Antigen wird zur Immunisierung von Mäusen und Herstellung monoklonaler Antikörper verwendet.

Die so erzeugten monoklonalen Antikörper werden vorzugsweise im ELISA, Immunoblot und RIPA auf Kreuzreaktivitäten mit verschiedenen Virus-lsolaten überprüft. Der geeignete monoklonale Antikörper besitzt idealerweise eine breite Kreuzreaktivität und erfaßt dadurch die gesamte Quasispezies eines Infizierten.

Wird kein monoklonaler Antikörper gewonnen, der alle Virusisolate erkennt, so werden Isolatspezifische monoklonale Antikörper hergestellt, die dann gezielt zur Therapie des im Einzelfall nachgewiesenen Isolats eingesetzt werden.

### 2. Herstellung von Radioimmunpharmaka auf der Basis von Wirtsrezeptormolekülen

Wie ebenfalls bereits oben geschildert. wurden inzwischen für eine ganze Reihe von Viren zelluläre Rezeptormoleküle identifiziert, die die Adsorption der Viren an die Oberfläche von Wirtszellen vermitteln. Die Methoden zur Identifizierung und Gewinnung solcher Rezeptormoloküle gehört zum Stand der Technik. Das Vorgehen soll im Beispiel 2 beschrieben werden. Der Rezeptor für HIV-1 und HIV-2 ist der CD4-Rezeptor, während das HCV wahrscheinlich an den LDL-Rezeptor bindet (Seipp et al. 1997).

### Beispiel 2:

Das betreffende Virus (z.B. HCV) wird gelelektrophoretisch aufgetrennt, so daß die (HC)viralen Antigene isoliert werden können. An Gewebeproben (aus Leber) und Zellkultursystemen werden dann Bindungsstudien durchgeführt werden und der gesuchte, für die Adsorption des Virus verantwortliche zelluläre Rezeptor identifiziert (Dorig et al. 1993. Treichel et al. 1997). Dieser Rezeptor wird mit etablierten Methoden isoliert (Suzuki et al. 1983) und das Bindungsepitop eingehender analysiert. indem die Bindung des Antigens an einzelnen Spaltprodukten analysiert wird. Das Rezeptormolekül wird schließlich kloniert (Bergelson et al. 1998) und so in großem Maßstab für die Konjugation mit einem Radioisotop und den therapeutischen Einsatz gemäß vorliegender Erfindung weiter verarbeitet.

### 3. Konjugation geeigneter Radionuklide

Die monoklonalen Antikörper oder Wirtsrezeptormoleküle werden mit einem Radionuklid (Alpha- bzw. Beta-Emitter) konjugiert. Die Konjugation von Radionukliden an Proteine ist hinreichend beschrieben und Stand der Technik (Übersicht in: Eckert und Kartenbeck 1996). Das Vorgehen soll im Beispiel 3 exemplarisch dargestellt werden, wenngleich verschiedene Methoden (z.B. Zalutzky et al. 1989) für bestimmte Konjugatkonstrukte mehr oder weniger geeignet sein können, so daß die methodischen Vorgehensweisen dem Einzelfall angepaßt und optimert werden sollten.

### Beispiel 3:

Geeignete monoklonale Antikörper oder Rezeptormoleküle werden zu therapeutischen Zwecken mit Hilfe der Chloramin T-Methode (Hunter und Greenwood 1962) nach der Anleitung von Eckert und Kartenbeck 1997 radioaktiv konjugiert. Hierzu wird ein Radioisotop kurzzeitig durch das von Chloramin T (N-Chlor-p-Toluen-4-sulfonamid, Na-Salz) in wässrigem Medium freigesetzte Hypochlorit oxidiert. Das stark elektrophile Radioisotop bindet dann in diesem Zustand vorzugsweise an die Benzolringe der im Protein enthaltenen aromatischen Aminosäuren. Zur Schonung der monoklonalen Antikörper bzw. Rezeptormoleküle wird diese Reaktion nach kurzer Inkubationszeit mit einem Überschuß an Bisulfit beendet, wobei sowohl das restliche Chloramin T als auch oxidierte. aber noch ungebundene Radioisotope reduziert und damit inaktiviert werden. Gelelektrophoretisch wird das mAk-Radioisotop-Konjugat schließlich isoliert und zur. vorzugsweise intravenösen, Verwendung mit entsprechenden Hilfsstoffen weiter aufgearbeitet. Weitere Verfahren stehen ebenfalls zur Verfügung (Harrison und Royle 1984, Zalutsky et al. 1989). Nach einem dieser Verfahren lassen sich z.B. die Konjugate a7) herstellen.

### Präklinische Untersuchungen

Zunächst zeigen in-vitro-Untersuchungen, ob und mit welcher Affinität die Radioimmunkonjugate über die Antigenbindungsstelle der monoklonalen Antikörper oder Wirtsrezeptormoleküle an die entsprechenden Epitope viraler, in die Zellmembran integrierter Proteine virusinfizierter Zellen binden und ob die Zellen durch die Radioisotopvermittelte Bestrahlung geschädigt werden. Diese Untersuchungen werden in geeigneten Zellkultursystemen durchgeführt.

An Nacktmäusen und anderen Tiermodellen können in den folgenden Schritten präklinische in-vivo-Untersuchungen zur Wirksamkeit der Radioimmuntherapie erfolgen. bevor die klinische Anwendung bei infizierten Patienten geprüft wird. Im Falle der HCV-Behandlung sind z.B. HCV-infizierte Schimpansen (Tabor et al. 1978, Walker et al. 1997) ein geeignetes Versuchssystem.

### Anwendung am Patienten: Therapeutische Voraussetzungen

In verschiedenen Phase-1-Studien (Dosiseskalationsstudien) sollen die maximal tolerierbaren Dosen (MTD) hinsichtlich Nebeneffekten. Pharmakokinetik und Immunogenität bestimmt werden. Durch die nachfolgenden klinischen Untersuchungen (Phase-ll- und -III-Studien) soll schließlich der Effekt einzelner Radioimmunpharmaka an kleinen Patientenkollektiven mit fortgeschrittener Erkrankung und an randomisierten Patientenkollektiven geprüft werden (Fiebig 1995).

Die kurze Halbwertszeit der Radionuklide (ob α- oder β-Strahler) macht eine zentrumsnahe Präparation bzw. schnellen Transport des Radioimmunkonjugats zum Therapeuten erforderlich. Sie ist aber insofern von Bedeutung, als längere Halbwertszeiten den behandelnden Patienten einer zu großen Strahlendosis über einen zu langen Zeitraum aussetzen. Voraussetzung für die Therapie von Patienten mit HIV. viralen Hepatitiden und ggf. anderen viralen Infektionen mit Hilfe von kurzlebigen Radionukliden ist daher die Einrichtung spezialisierter interdisziplinärer Zentren, in denen neben einer fachgerechten Therapie der Infizierten auch die zeitgerechte Applikation des Radioimmunkonjugates unter strahlenschutzrechtlichen Aspekten gewährleistet ist.

Voraussetzung einer erfolgreichen Therapie kann auch die prätherapeutische Verminderung der Viruslast sein, die durch Vorbehandlung des Patienten mit antiviralen bzw. antiretroviralen Präparaten erzielt werden kann. Dadurch gelangt eine höhere Dosis des Radioimmunpharmakons an die Virusreplizierenden Zellen, wodurch der therapeutische Effekt verbessert wird. Andernfalls würde das Radioimmunpharmakon möglicherweise von freien Viruspartikeln abgefangen und die cytotoxische Wirkung vermindert werden. Bei der HCV-Infektion kann eine Vorbehandlung mit IFN-α oder Ribavirin erfolgen. Das kann als Mono- oder auch als Kombinationstherapie geschehen.

Vor Applikation eines auf ¹³¹I basierenden Präparats muß außerdem eine Schilddrüsendiagnostik und Schilddrüsenblockade nach üblichem Schema erfolgen.

Für die Radioimmuntherapie ist eine mehrtägige stationäre Unterbringung erforderlich. um den Patienten bis zum Abklingen der Strahlung von der Umgebung abzuschirmen. Das Radioimmunpharmakon wird peripher oder zentralvenös als Bolus, Kurzinfusion oder Dauertherapie über mehrere Tage mit einer Dosis von 25 - 300 mCi. vorzugsweise 50 - 300, besonders bevorzugt 100-200 mCi. appliziert. Die Gabe erfolgt einmalig oder in Form von Zyklen im mehrwöchigen Abstand. Gegebenenfalls ist bei bestehender Überempfindlichkeit gegenüber dem monoklonalen Antikörper oder gegenüber dem Rezeptormolekül unmittelbar vor Applikation des Präparates eine Vorbehandlung mit einem Glukokortikoid. einem Antihistaminikum und/oder einem H₂-Antagonisten erforderlich (Lorenz 1994).

Als eine besonders vorteilhafte Ausgestaltung der Erfindung erweist sich die Verwendung humanisierter bzw. humaner monoklonaler Antikörper. wodurch die Immunogenität muriner mAk-Konjugate umgangen werden kann. Diese kann sich andererseits aber auch als sinnvoll erweisen, wenn es gilt, das Immunsystem zusätzlich gegenüber der Virus-Infektion zu sensibilisieren.

Eine weitere vorteilhafte Ausgestaltung betrifft die Verwendung von Fragmenten monoklonaler Antikörper oder Zellrezeptoren als immunologisch wirksame Komponente der Radioimmurkonjugate. da durch die geringere Molekülgröße eine bessere Gewebegängigkeit und Penetrationsfähigkeit durch die Blut-Him-Schranke erzielt werden kann.

Eine enorme präventivmedizinische Bedeutung kommt der Radioimmuntherapie viraler Infektionen insbesondere in den Fällen zu, in denen es darum geht, durch eine präventive Viruseliminierung onkologische Erkrankungen zu verhindern bzw. zu bekämpfen, wie sie beispielsweise als Folge von HIV-, HTLV-1, HTLV-2, HHV8-, EBV-, HCV- und HBV-Infektionen bekannt sind.

Wenngleich Beta- und Alpha-Strahler bereits in die Radioimmuntherapie maligner Erkrankungen Eingang gefunden haben, so handelt es sich im Gegensatz dazu hier nicht nur um eine völlig neue Indikation für Radioimmunpharmaka allgemein, sondern um einen grundsätzlich anderen therapeutischen Ansatz und Anspruch für sehr spezifisch konstruierte antivirale Präparate mit einem jeweils sehr genau definierten Einsatzgebiet. Während bei der Radioimmuntherapie maligner Erkrankungen monoklonale Antikörper gegen *zelleigene, tumorspezifische* Proteine verwendet werden. wurden bei den hier beschriebenen Radioimmunkonjugaten monoklonlale Antikörper bzw. deren Fragmente oder andere Proteine bzw. Peptide mit therapeutischer Zielsetzung speziell gegen *virale* (d.h. nicht zelleigene!) und virusinduzierte (d.h. nicht Zelltypspezifische) Proteine beschrieben.

### Literatur

1. Alter HJ. Purcell RH. Holland PV et al.: Transmissible agent in non-A. non-B hepatitis. Lancet 1978: 1: 459-463.
2. Berg T und Hopf U: Interferontherapie der HCV-Infektion: In: Häussinger D. Niederau C: Hepatitis C. Blackwell Wissenschafts-Verlag Berlin 1997: 229-267.
3. Bergelson JM. Krithivas A. Celi L. et al.: The murine CAR homolog is a receptor for coxsackie B viruses and adenoviruses. J Virol 1998; 72 (1): 415-419.
4. Berater W: Herstellung zehn monoklonaler Antikörper gegen das Affen-Immundefizienzvirus SIVagmTYO-7 aus der Familie der Retroviren. Dissertation. Hannover 1990.
5. Blumenthal RD. Sharkey RM, Haywood L, et al.: Targeted therapy of athymic mice bearing GW-39 human colonic cancer micrometastases with ¹³¹J-labeled monoclonal antibodies. Cancer Res 1992; 52: 6036-6044.
6. Blumenthal RD. Sharkey RM, Natale AM, et al.: Comparison of equitoxic radioimmunotherapy and chemotherapy in the treatment of human colonic cancer xenografts. Cancer Res 1994: 54: 142-151.
7. Böker. KHW und Manns MP: Was bringt die Interferontherapie bei der chronischen Hepatitis. Internist 1997; 38: 1191-1203.
8. Boulan ER. Pendergast M: Polarized distribution of viral envelope proteins in the plasma membrane of infected epithelial cells. Cell 1980: 20 (1): 45-54.
9. Brodt HR, Helm EB. Kamps BS: AIDS 1997. Diagnostik und Therapie. Steinhäuser Verlag. Wuppertal 1997: 81-117.
10. Byrn RA Sekigawa l. Chamow SM. et al.: Characterization of in vitro inhibition of human immunodeficiency virus by purified recombinant CD4. J Virol. 1989; 63(10): 4370-4375.
11. Choo QL. Kuo G, Weiner AJ. et al.: Isolation of a cDNA clone derived from a blood-borne non-A. non-B viral hepatitis genome. Science 1989; 244: 359-362.
12. Clapham PR. Weber JN. Whitby D. et al.: Soluble CD4 blocks the infectivity of diverse strains of HIV and SIV for T cells and monocytes but not for brain and muscle cells. Nature 1989: 337(6205): 368-370.
13. Ciampor F. Sidorenko EV. Taikova NV: Ultrastructural localization by immunoperoxidase techniques of influenza virus antigens in abortive infection of L cells. Acta Virol 1981; 25 (6): 381-389.
14. Conill C. Alsina M, Verger E. et al.: Radiation therapy in AIDS-related cutaneous Kaposi's sarcoma. Dermatology 1997:195(1): 40-42.
15. Chu CM, Liaw YF: Natural history of chronic hepatitis B virus infection: an immunopathological study. J Gastroenterol Hepatol 1997; 12 (9-10): 218-222.
16. Dalgleish AG. Beverly PC, Clapham PR et al.: The CD4 (T4) antigen is an essential component of the receptor for the AIDS retrovirus. Nature 1984; 312 (5996): 763-767.
17. Darai G. et al.: Lexikon der Infektionskrankheiten des Menschen. Springer. Heidelberg 1997
18. Deen KC, McDougal JS, Inacker R et al.: A soluble form of CD4 (T4) protein inhibits AIDS virus infection. Nature 1988; 331(6151): 82-84.
19. Dennin RH, Beyer A: Application of scanning electron microscopy (SEM) and microbead techniques to study the localization of p24 and p18 antigens of HIV-1 on the surface of HIV-1-infected H9-lymphocytes. J Microsc 1991;164 ( Pt 1): 53-60.
20. Dorig RE. Marcil A, Chopra A. et al.: The human CD46 molecule is a receptor for measles virus (Edmonstron strain). Cell 1993; 75 (2): 295-305.
21. Desportes I. Bonnet D, Nicol 1. et al.: Expression of HIV antigens at the surface of infected T4 cells: immunoelectron microscopic evidence of an immunogenic phase prior to the viral release. AIDS Res Hum Retroviruses 1989: 5 (1): 107-114.
22. Dudhane A. Wang ZQ. Orlikowsky T. et al.: AIDS patient monocytes target CD4 T cells for cellular conjugate formation and deletion through the membrane expression of HIV-1 envelope molecules. AIDS Res Hum Retroviruses 1996; 12 (10): 893-899.
23. Eckert WA und Kartenberg J: Proteine: Standardmethoden der Molekular- und Zellbiologie. Springer, Heidelberg 1997: 6-12, 237-240.
24. Erickson JW and Burt SK: Structural mechanisms of HIV drug resistance. Annu Rev Pharmacol Toxicol 1996: 36: 545-571.
25. Farci P, Shimoda A. Wong D. et al.: Prevention of hepatitis C virus infection in chimpanzees by hyperimmune serum against the hypervariable region 1 of the envelope 2 protein. Proc Natl Acad Sci USA 1996; 93 (26): 15394-15399.
26. Feremans WW. Huygen K. Menu R, et al.: Fifty cases of human immunodeficiency virus (HIV) infection: immunoultrastructural study of circulating lymphocytes. J Clin Pathol 1988: 41 (1): 62-71.
27. Fetzer I.C.. Bergter W, Ramadori G: Kombinationstherapie mit INF-α2a und Ribavirin nach erfolgloser IFN-α2a-Monotherapie bei Patienten mit chronischer HCV-Infektion. Göttinger Erfahrung. Z Gastroenterol 1997; 35: 419-433.
28. Fiebig HH: Durchführung von klinischen Studien. In Zeller WJ. Zur Hausen H (Hrsg): Onkologie, Grundlagen, Diagnostik, Therapie, Entwicklungen, Ecomed, Landsberg/Lech 1995: IV-2: 1-5.
29. Fingeroth JD; Weis JJ; Tedder TF et al.: Epstein-Barr virus receptor of human B lymphocytes is the C3d receptor CR2. Proc Natl Acad Sci U S A 1984: 81 (14): 4510-4514.
30. Follea G. Herve P. Andreu G. et al.: Viral attenuation of labile blood products. Transfus Clin Biol 1996; 3 (2): 113-123.
31. Fultz PN, Schwiebert RS Su L. et al.: Effects of total lymphoid irradiation on SIV-infected macaques. AIDS Res Hum Retroviruses 1995; 11(12): 1517-1527.
32. Gaubin M. Autiero M, Houlgatte R, et al.: Molecular basis of T lymphocyte CD4 antigen functions. Eur J Clin Chem Clin Biochem 1996;34(9): 723-728.
33. Gerber MA, Sells MA, Chen ML, et al.: Morphologic, immunohistochemical, and ultrastructural studies of the production of hepatitis B virus in vitro. Lab Invest 1988; 59 (2): 173-180.
34. Glick BR und Pasternak JJ: Molekulare Biotechnologie. Spektrum Akademischer Verlag Heidelberg 1995: 91-164.
35. Harrison A. Royle L: Preparation of a 211At-IgG conjugate which is stable in vivo. Int J Appl Radiot Isot 1984; 35(11): 1005-1008.
36. Hughey PG, Compans RW, Zebedee SL, et al.: Expression of the influenza A virus M2 protein is restricted to apical surfaces of polarized epithelial cells. J Virol 1992; 66 (9): 5542-5552.
37. Hunter WM, Greenwood FC: Preparation of iodine-131 labeled human growth hormone of high specific activity. Nature 1962; 194: 495-496.
38. Imrie A. Beveridge A. Genn W: Transmission of human immunodeficiency virus type l resistant to nevirapine and zidovudine. Sydney Primary HIV Infection Study Group. J Infect Dis 1997;175(6): 1502-1506.
39. Ikuta K. Morita C. Miyake S. et al.: Expression of human immunodeficiency virus type l (HIV-1) gag antigens on the surface of a cell line persistently infected with HIV-1 that highly expresses HIV-1 antigens. Virology 1989; 170 (2): 408-417.
40. Jones DH. Sakamoto K. Vorce RL, Howard BJ: DANN mutagenesis and recombination. Nature 1990: 344: 793-794.
41. Kaito M. Watanabe S. Tsukiyama-Kohara K, et al.: Hepatitis C virus particle detected by immunoelectron microscopic study. J Gen Virol 1994: 75 ( Pt 7): 1755-1760.
42. Kaminski MS. Zasadny KR. Francis IR, et al.: Radioimmunotherapy of B-cell lymphoma with [¹³¹J]anti-B1 (anti-CD20) antibody. N Engl J Med 1993; 329: 459-465.
43. Kaplan G. Totsuka A. Thompson P. et al.: Identification of a surface glycoprotein on African green monkey kidney cells as a receptor for hepatitis A virus. EMBO J 1996: 15 (16): 4282-4296.
44. Kauffmann G, Moser E. Sauer R: Radiologie. Grundlagen der Radiodiagnostik, Radiotherapie und Nuklearmedizin. Urban und Schwarzenberg München 1996.
45. Kilby JM et al., Potent suppression of HIV-1 replication in humans by T-20, a peptide inhibitor of gp41-mediated virus entry.Nat. Med. 4(11): 1302-1307 (1998),
46. Kohama MT, Cardenas JM, Seto JT: Immunoelectron microscopic study of the detection of the glycoproteins of influenza and Sendai viruses in infected cells by the immunoperoxidase method. J Virol Methods 1981;3 (5): 293-301.
47. Kozak RW, Atcher RW, Gansow OA, et al.: Bismuth-212-labeled anti-Tac monoclonal antibody: alpha-particle-emitting radionuclides as modalities for radioimmunotherapy. Proc Natl Acad Sci U S A 1986: 83(2): 474 - 478.
48. Krain J. Dieckmann KP: Treatment of tsticular seminoma in patients with HIV infection, Report of two cases. Eur Urol 1994: 26(2): 184-186.
49. Lentz TL, Benson RJ, Klimowicz D, et al.: Binding of rabies virus to purified Torpedo acetylcholine receptor. Brain Res. 1986; 387 (3): 211-219.
50. Leon-Monzon ME, Illa I, Dalakas MC: Expression of poliovirus receptor in human spinal cord and muscle, Ann N Y Acad Sci 1995; 753: 48-57.
51. Liddell E und Weeks 1: Antikörper-Techniken, Spektrum Akademischer Verlag, Heidelberg 1996.
52. Liebowitz D, Wang D, Kieff E: Orientation and patching of the latent infection membrane protein encoded by Epstein-Barr virus, J Virol 1986; 58 (1): 233-237.
53. Lorenz R: Bildgebende Verfahren. ln: Classen M, Diehl V, Kochsiek K: Innere Medizin, Urban & Schwarzenbeck München 1994 (3. Auflage): 39-63.
54. Lotz R Rosler HP, Zapf S, et al.: Radiotherapy in HIV positive patients. Fortschr Med 1990: 108(2): 35-36,39.
55. Macklis RM, Kinsey BM. Kassis AI, et al.: Radioimmuntotherapy with alpha-particle-emitting immunoconjugates. Science 1988; 240(4855): 1024-1026.
56. Maier KP: Pathologie der Hepatitis C. In: Häussinger D. Niederau C: Hepatitis C. Blackwell Wissenschafts-Verlag Berlin 1997: 149-163.
57. Manns MP und Rainbusch EG: Extrahepatische Manifestationen und Autoimmunität bei HCV-Infektion. In: Häussinger D. Niederau C: Hepatitis C. Blackwell Wissenschafts-Verlag Berlin 1997: 165-182.
58. Martin CE und Scheinbach S: Expression of proteins encoded by foreign genes in *Saccharomyces cerevisiae.* Biotech Adv 1989; 7: 155-185.
59. Moser E: Nuklearmedizin. In Kauffmann G. Moser E und Sauer R: Radiologie. Urban und Schwarzenberg, München 1996: 253-283.
60. Muster T, Steindl F, Purtscher M, et al.: A conserved neutralizing epitope on gp41 of human immunodeficiency virus type 1. J Virol 1993; 67(11): 6642-6647.
61. Nakajima A, Hijikata M, Yoshikura H, et al.: Charakterization of longterrn cultures of hepatitis C virus. J Virol 1996; 70: 3325-3329.
62. Ng Ml. Choo Wk, Ho Yl: Detection of flavivirus antigens in purified infected Vero cell plasma membranes. J Virol Methods 1992; 39 (1-2): 125-138.
63. Nishimura H, Sugawara K, Kitame F, et al.: Attachment of influenza C virus to human erythrocytes. J Gen Virol 1988; 69 (Pt 10): 2545-2553.
64. Payne HR; Storz J; Henk WG: Bovine coronavirus antigen in the host cell plasmalemma. Exp Mol Pathol 1990; 53 (2): 152-159.
65. Peters JH und Baumgarten H: Monoklonale Antikörper. Herstellung und Charakterisierung. Springer, Berlin 1990 (2. Auflage).
66. Petry et al.: Hepatozelluläres Karzinom bei HCV-Infektion. In: Häussinger D. Niederau C: Hepatitis C. Blackwell Wissenschafts-Verlag Berlin 1997: 183-219.
67. Plettenberg A, Janik I, Kolb H, et al.: Local therapy measures in HIV-associated Kaposi's sarcoma with special reference to fractionated radiotherapy. Strahlenther Onkol 1991: 167(4):208-213.
68. Pozzetto B, Bourlet T, Grattard F et al.: Structure, genomic organization, replication and variability of hepatitis C virus. Nephrol Dial Transplant 1996; 11 (Suppl 4): 2-5.
69. Press OW, Eary JF, Appelbaum FR et al.: Radiolabeled-antibody therapy of B-cell lymphoma with autologous bone marrow support. N Engl J Med 1993; 329: 1219-1224,
70. Press OW, Eary JF, Appelbaum FR, et al.: Phase II trial of ¹³¹J-B1(anti-CD20) antibody therapy with autologous stem cell transplantation for relapsed B-cell lymphoma. Lancet 1995: 346: 336-340.
71. Press OW, Eary JF, Appelbaum FR, et al.: Treatment of relapsed B-cell lymphomas with high dose radioimmunotherapy and bone marrow transplantation. In Goldenberg DM.(ed.): Cancer Therapy with Radiolabeled Antibodies. Boca Raton, CRC Press 1995: 229-237.
72. Purcell R: The hepatitis C virus: overview. Hepatology 1997;26 (3 Suppl 1): 11S-14S.
73. Reichard O, Yun ZB, Sönnerborg A, et al.: Hepatitis C viral RNA titers in serum prior to, during, and after oral treatment with ribavirin for chronic hepatitis C. J Med Virol 1993; 41: 99-102.
74. Reiners C: Zum Krebs- und genetischen Risiko nach Radioiodtherapie der Hyperthyreose. Der Nuklearmediziner 1997: 5 (20): 331-334.
75. Revilla-Monsalve C, Hernandez-Jauregui P, Silva-Lemoine E: Immunoperoxidase cell surface localization of rabies virus antigen in tissue cultures at a low viral multiplicity. Arch Invest Med (Mex) 1985; 16 (1): 11-17.
76. Rosa D, Campagnoli S, Moretto C, et al.: A quantitative test to estimate neutralizing antibodies to the hepatitis C virus: cytofluorometric assessment of envelope glycoprotein 2 binding to target cells. Proc Natl Acad Sci USA 1996; 93 (5): 1759-1763.
77. Rusche JR, Lynn DL, Robert-GuroffM, et al.: Humoral immune response to the entire human immunodeficiency virus envelope glycoprotein made in insect cells. Proc Natl Acad Sci USA 1987; 84 (19): 6924-6928.
78. Sabri S, Richelme F, Pierres A et al.: Interest of image processing in cell biology and immunology. J Immunol Methods 1997: 208 (1): 1-27.
79. Saito T, Kamimura T, Ishibashi M, et al.: Electron microscopic study of hepatitis B virusassociated antigens on the infected liver cell membrane in relation to analysis of immune target antigens in chronic hepatitis B. Gastroenterol Jpn 1992; 27 (6): 734-744.
80. Santolini E, Pacini L, Fipaldini C, et al.: The NS2protein of hepatitis C virus is a transmembrane polypeptide. J Virol 1995; 69 (12): 7461-7471.
81. Saran F, Adamietz IA, Mose S, et al.: The value of conventionally fractionated radiotherapy in the local treatment of HIV-related Kaposi's sarcoma. Strahlenther Onkol 1995: 171(10): 594-599.
82. Saran FH, Adamietz IA, Thilmann C, et al.: HIV-associated cutaneous Kaposi's sarcoma - palliative local treatment by radiotherapy. Acta Oncol 1997; 36(1): 55-58.
83. Sattentau QJ, Clapham PR, Weiss RA, et al.: The human and simian immunodeficiency viruses HIV-1, HIV-2 and SIV interact with similar epitopes on their cellular receptor, the CD4 molecule. AIDS 1988: 2(2): 101-105.
84. Sauer R: Strahlenbiologie. In: Kauffmann G. Moser E und Sauer R: Radiologie. Urban und Schwarzenberg, München 1996: 31-80.
85. Sauer R: Strahlentherapie und Onkologie fiir MTA-R. Urban und Schwarzenberg, München 1998.
86. Scheidegger C, Heinrich B, Popescu M, et al.: HIV associated lymphomas. Dtsch Med Wochenschr 1991; 116(30):1129-1135.
87. Schlehofer JR, Hampl H, Habermehl KO: Differences in the morphology of herpes simplex virus infected cells: 1. Comparative scanning and transmission electron microscopic studies on HSV-1 infected HEp-2 and chick embryo fibroblast cells. J Gen Virol 1979; 44 (2): 433-442.
88. Schvarcz R, Yun ZB, Sonnerborg A et al.: Combined treatment with interferon alpha-2b and ribavirin for chronic hepatitis C in patients with a previous non-response or nonsustained responseto interferon alone. J Med Virol 1995; 46 (1): 43-47.
89. Seipp S, Mueller HM, Pfaff E, et al.: Establishment of persistent hepatitis C virus infection and replication in vitro. J Gen Virol. 1997; 78 (Pt10): 2467-2476.
90. Selby MJ, Choo QL, Berger K,et al.: Expression, identification and subcellular localization of the proteins encoded by the hepatitis C viral genome. J Gen Virol 1993: 74 (Pt 6): 1103-1113.
91. Shafren DR, Williams DT, Barry RD: A decay-accelerating factor-binding strain of coxsackievirus B3 requires the coxsackievirus-adenovirus receptor protein to mediate lytic infection of rhabdomyosarcoma cells. J Virol 1997; 71 (12): 9844-9848.
92. Shimotohno K, Tanji Y, Hirowatari Y et al.: Processing of the hepatitis C virus precurser protein. J Hepatol 1995; 22 (Suppl.1): 87-92.
93. Stirling JW: Immuno- and affinity probes for electron microscopy: a review of labeling and preparation techniques. J Histochem Cytochem 1990; 38 (2): 145-157.
94. Su SB et al.: T20/DP178, an ectodomain peptide of human immunodeficiency virus type 1 gp41, is an activator of human phagocyte N-formyl peptide receptor. Blood 93(11):3885-3892 (1999)
95. Suzuki Y, Suzuki T, Matsumoto M: Isolation and characterizationof receptor sialoglycoprotein for hemagglutinating virus of Japan (Sendai virus) from bovine erythrocyte membrane. J Biochem (Tokyo) 1983; 93 (6): 1621-1633.
96. Swift PS: The role of radiation therapy in the management of HIV-related Kaposi's sarcoma. Hematol Oncol Clin North Am 1996; 10(5): 1069-1080.
97. Tabor E, Gerety RJ, Drucker JA: Transmission of non-A, non-B hepatitis from man to chimpanzee. Lancet 1978; 1: 463-466.
98. Timar J, Nagy K, Lapis K: Morphologic and immunoelectronmicroscopic identification of human T-cell lymphotropic virus type III (HTLV-III). Histol Histopathol 1986:1 (1): 43-47.
99. Treichel U, Meyer zum Büschenfelde, Dienes H-P et al.: Receptor-mediated entry of hepatitis B virus particles into liver cells. Arch Virol 1997; 142: 493-498.
100. Turkington TG. Zalutzky MR, Jaszczak RJ, Garg P, Vaidynathan G. Coleman RE. Measuring astatine-21 distributions with SPECT. Phys Med Biol 1993; 38: 1121-1130.
101. Vento S, Concia E, Ferraro T: Lack of sustained efficiacy of interferon in patients with chronic hepatitis C. N Engl J Med 1996; 334: 1479.
102. Walker CM: Comparative features of hepatitis C virus infection in humans and chimpanzees. Springer Semin Immunopathol 1997;19 (1): 85-98.
103. Watanabe M, Reimann KA, DeLong PA, et al.: Effect of recombinant soluble CD4 in rhesus monkeys infected with simian immunodeficiency virus of macaques. Nature 1989; 337(6204): 267-272.
104. Westaway EG, Goodman MR: Variation in distribution of the three flavivirus-specific glycoproteins detected by immunofluorescence in infected Vero cells. Arch Virol 1987; 94 (3-4): 215-228.
105. Whitbeck JC, Peng C, Lou H, et al.: Glycoprotein D of herpes simplex virus (HSV) binds directly to HVEM, a member of the tumor necrosis factor receptor superfamily and a mediator of HSV entry. J Virol 1997; 71 (8): 6083-6093.
106. Wilcox G und Studnicka GM: Expression of foreign proteins in microorganisms. Biotech Appl. Biochem 1988: 10: 500-509.
107. Xu Y, Conway B Montaner JS, et al.: Effect of low-dose gamma radiation of HIV replication in human peripheral blood mononuclear cells. Photochem Photobiol 1996: 64(2): 238-241.
108. Zalutzky MR und Bigner DD: Radioimmunotherapy with α-particle emitting radioimmunoconjugates. Acta Oncol 1996; 35(3): 373 - 379.
109. Zalutzky MR, Garg PK, Friedmann HS et al.: Labeling monoclonal antibodies and F(ab')2 fragments with the alpha-particle emitting nuclide astatine-211: preservation of immurioreactivity and in vivo localizing capacity. Proc Natl Acad Sci U S A 1989; 86(18): 7149-7153.
110. Zilpert A und Roggendorf M: Molekularbiologie des Hepatitis-C-Virus. In: Häussinger D, Niederau C: Hepatitis C. Blackwell Wissenschafts-Verlag Berlin 1997: 13-51.
111. Zweit J: Radionuclides and carrier molecules for therapy. Phys Med Biol 1996; 41: 1905-1914.

## Patentansprüche

1. Verwendung eines Radioimnunkonjugats (RIK) zur Herstellung eines Therapeutikums für die Behandlung von viralen Infektionen und von durch diese induzierten Tumoren,
**dadurch gekennzeichnet, dass** das Konjugat
als immunologisch wirksame Komponente
a) ein Rezeptormolekül bzw. dessen Fragment mit Affinität zu einem Epitop der auf der Plasmamembran infizierter Zellen exprimierten viralen Strukturproteine oder
b) ein durch Mutagenese modifiziertes Fragment des zellulären Rezeptormoleküls mit Affinität zu einem Epitop der auf der Plasmamembran infizierter Zellen exprimierten viralen Struktuiproteine
und als radioaktive Komponente
c) einen Alpha-Strahler oder einen Beta-Strahler enthält

2. Verwendung eines Radioimmunkonjugats (RIK) zur Herstellung eines Therapeutikums für die Behandlung von viralen Infektionen und von durch diese induzierten Tumoren, **dadurch gekennzeichnet, dass** das Konjugat
als immunologisch wirksame Komponente
a) einen monoklonalen Antikörper bzw. dessen Antigen bindendes Fragment gegen ein auf der Plasmamembran mit Virus infizierter Zellen exprimiertes virales oder Virus induziertes Antigen
und als radioaktive Komponente
b) einen Alpha-Strahler oder einen Beta-Strahler enthält,
und weiterhin **dadurch gekennzeichnet, dass**
c) das Therapeutikum für eine HIV-Infektion für die Verabreichung im Anschluß an eine oder während einer antiretroviralen Therapie wie der Standardtripeltherapie zubereitet wird, und/oder
d) das Therapeutikum für eine HBV-, HCV-, oder HDV-Iafektion für die Verabreichung im Anschluß an eine oder während einer IFN-α-Mono- oder Kombinationstherapie mit Ribavirin zubereitet wird,
mit der Einschränkung, dass das RIK kein Therapeutikum für die Behandlung von HTV-Infektionen und von durch diese induzierten Tumoren ist, wenn der monoklonale Antikörper bzw. dessen Fragment als radioaktive Komponente einen Beta-Strahler enthält.

3. Verwendung nach Patentanspruch 1 oder. 2, **dadurch gekennzeichnet, dass** die virale Infektion eine HIV-, HBV-, HCV-, HDV-, HTLV-, CMV-, EBV- oder HHV8-Infektion ist

4. Verwendung nach Patentanspruch 3, **dadurch gekennzeichnet, dass** die Infektion eine HIV-Infektion, insbesondere eine Infektion mit HIV-1, HIV-2 oder HIV-3, eine HBV-Infektion, eine HCV-Infektion oder eine HTLV-Infektion, insbesondere eine Infektion mit HTLV-1 oder HTLV-2, ist

5. Verwendung nach einem der Patentansprüche 1, 3 oder 4, **dadurch gekennzeichnet, dass** das Radioimmunkonjugat als immunologisch wirksame Komponente den CD4-Rezeptor bzw. ein Fragment davon mit Affinität zu einem Epitop von gp120 von HIV-1 und/oder HIV-2 ist.

6. Verwendung nach einem der Patentansprüche 1, 3 oder 4, **dadurch gekennzeichnet, dass** das Radioimmunkonjugat als immunologisch wirksame Komponente den LDL-Rezeptor oder einen anderen Rezeptor mit Affinität zu einem Epitop auf dem Oberflächenglykoprotein E1 bzw. E2 des HCV bzw. ein Fragment von diesen Rezeptoren ist.

7. Verwendung nach einem der Patentansprüche 1, 3 oder 4, **dadurch gekennzeichnet, dass** das Radioimmunkonjugat als immunologisch wirksame Komponente den ASGPR-Rezeptor oder einen anderen Rezeptor mit Affinität zu Oberflächenglykoproteinen des HBV bzw. ein Fragment von diesen Rezeptoren ist.

8. Verwendung nach einem der vorhergehenden Patentansprüche, **dadurch gekennzeichnet, dass** das Radioimmunkonjugat für die ein- oder mehrmalige intravenöse Verabreichung von einer Gesamtkörperdosis von 25 bis 300 mCi unter ein- bis mehrtägiger Strahlenabschirmung des Patienten von der Umgebung zubereitet wird.

9. Verwendung nach einem der vorhergehenden Patentansprüche, **dadurch gekennzeichnet, dass** der α-bzw. β-Strahler eine Halbwertszeit von 10 Tagen und weniger hat.

10. Verwendung nach Patentanspruch 9, **dadurch gekennzeichnet, dass** der α-Strahler Wismut-212 und/oder Wismut-213 und/oder Astat-211 und/oder Radium-223 und/oder Actinium-225 und der β-Strahler ¹³¹I und/oder ⁸⁹Sr und/oder ¹⁷⁷Lu und/oder ¹⁸⁶Lu und/oder ¹⁸⁶Re und/oder ¹⁸⁸Re und/oder ¹⁰⁵Rh und/oder ⁴⁷Sc und/oder ¹⁵³Sm und/oder ¹⁴⁹Tb ist bzw. enthält.

11. Verwendung nach einem der vorhergehenden Patentansprüche, **dadurch gekennzeichnet, dass**
a) das Therapeutikum füreine HIV-Infektion für die Verabreichung im Anschluß an eine oder während einer antiretroviralen Therapie wie der Standardtripeltherapie zubereitet wird, und/oder
b) das Therapeutikum fiir eine HBV-, HCV-, oder HDV-Ihfektion für die Verabreichung im Anschluß an eine oder während einer IFN-α-Mono- oder Kombinationstherapie mit Ribavirin zubereitet wird, und/oder
c) das Therapeutikum für die Verabreichung vor, während oder nach einem operativen Eingriff, insbesondere Transplantation einer infolge viraler Hepatitis zirrhotisch umgebauten Leber oder Resektion eines durch eine virale Hepatitis induzierten hepatozellulären Karzinoms, zubereitet wird, und/oder
d) das Therapeutikum für die Verabreichung unter dem Schutz einer Stammzelltransplantation zubereitet wird, und/oder
e) das Therapeutikum für die Verabreichung nach, vor oder gleichzeitig mit einer Verabreichung einer immunologischen Komponente, wie in Patentanspruch 1 bzw. 2 definiert, ohne radioaktive Komponente zubereitet wird.

12. Kit, umfassend eine oder mehrere immunologische Komponenten, wie in Patentanspruch 1 bzw. 2 definiert, und eine chromatographische Säule.

13. Kit nach Patentanspruch 12, weiterhin umfassend ein Lösungsmittel für die immunologische(n) Komponente(n) sowie ggf. ein Kopplungsmittel wie Chloramin T zum Herstellen des Konjugats (RIK).

## Claims

1. A use of a radioimmune conjugate (RIC) to produce a therapeutic agent for treatment of viral infections and tumors induced by them,
**characterized in that** the conjugate contains
as the immunologically active component
a) a receptor molecule and/or a fragment thereof having an affinity for an epitope of the viral structural proteins expressed on the plasma membrane of infected cells or
b) a fragment of the cellular receptor molecule modified by mutagenesis and having an affinity for an epitope of the viral structural proteins expressed on the plasma membrane of infected cells,
and as the radioactive component
c) an alpha-emitter or a beta-emitter.

2. The use of a radioimmune conjugate (RIC) to produce a therapeutic agent for treatment of viral infections and tumors induced by them,
**characterized in that** the conjugate contains
as the immunologically active component
a) a monoclonal antibody and/or its antigen binding fragment against a viral or virus-induced antigen expressed on the plasma membrane of virus-infected cells,
and as the radioactive component
b) an alpha-emitter or a beta-emitter,
and also **characterized in that**
c) the therapeutic agent is prepared for an HIV infection for administration following or during an antiretroviral therapy such as standard triple therapy, and/or
d) the therapeutic agent for an HBV, HCV or HDV infection is prepared for administration following or during an IFN-∀ monotherapy or combination therapy with ribavirin,
with the restriction that the radioimmune conjugate is not a therapeutic agent for treatment of HIV infections and tumors induced by these infections if the monoclonal antibody and/or fragment thereof contains a beta-emitter as the radioactive component.

3. The use according to Patent Claim 1 or 2, **characterized in that** the viral infection is an HIV, HBV, HCV, HDV, HTLV, CMV, EBV or HHV8 infection.

4. The use according to Patent Claim 3, **characterized in that** the infection is an HIV infection, in particular an infection with HIV-1, HIV-2 or HIV-3, an HBV infection, an HCV infection or an HTLV infection, in particular an infection with HTLV-1 or HTLV-2.

5. The use according to one of Patent Claims 1, 3 or 4, **characterized in that** the radioimmune conjugate has as the immunologically active component the CD-4 receptor and/or a fragment thereof having an affinity for an epitope of gp120 of HIV-1 and/or HIV-2.

6. The use according to one of Patent Claims 1, 3 or 4, **characterized in that** the radioimmune conjugate has as the immunologically active component the LDL receptor or another receptor having an affinity for an epitope on the surface glycoprotein E1 and/or E2 of HCV and/or a fragment of these receptors.

7. The use according to one of Patent Claims 1, 3 or 4, **characterized in that** the radioimmune conjugate contains as the immunologically active component the ASGPR receptor or another receptor having an affinity for surface glycoproteins of HBV and/or a fragment of these receptors.

8. The use according to one of the preceding patent claims, **characterized in that** the radioimmune conjugate is prepared for single or multiple intravenous administration of a whole-body dose of 25 to 300 mCi with radiation shielding of the patient from the environment for one or more days.

9. The use according to one of the preceding patent claims, **characterized in that** the α- and/or β-emitter has a half-life of ten days or less.

10. The use according to Patent Claim 9, **characterized in that** the α-emitter is and/or contains bismuth-212 and/or bismuth-213 and/or astate-211 and/or radium-223 and/or actinium-225, and the β-emitter is and/or contains ¹³¹I and/or ⁸⁹Sr and/or ¹⁷⁷Lu and/or ¹⁸⁶Lu and/or ¹⁸⁶Re and/or ¹⁸⁸Re and/or ¹⁰⁵Rh and/or ⁴⁷Sc and/or ¹⁵³Sm and/or ¹⁴⁹Tb.

11. The use according to one of the preceding patent claims, **characterized in that**
a) the therapeutic agent is prepared for an HIV infection for administration following or during an antiretroviral therapy such as the standard triple therapy, and/or
b) the therapeutic agent for an HBV, HCV or HDV infection is prepared for administration following or during an IFN-∀ monotherapy or combination therapy with ribavirin,
c) the therapeutic agent is prepared for administration before, during or after a surgical procedure, in particular transplantation of a liver that has become cirrhotic due to viral hepatitis or resection of a hepatocellular carcinoma induced by viral hepatitis, and/or
d) the therapeutic agent is prepared for administration under the protection of a stem cell transplantation, and/or
e) the therapeutic agent is prepared for administration before, after or concurrently with administration of an immunologic component as defined in Patent Claim 1 and/or 2, without the radioactive component.

12. A kit comprising one or more immunologic components, as defined in Patent Claim 1 and/or 2, and a chromatography column.

13. The kit according to Patent Claim 12, additionally comprising a solvent for the immunologic component(s) plus optionally a coupling agent such as chloramine-T for producing the conjugate (RIC).

## Revendications

1. Mise en oeuvre d'une substance de conjugaison radio-immunologique (SCRI) en vue de l'élaboration d'un agent thérapeutique pour le traitement d'infections virales et de tumeurs induites par ces dernières,
**caractérisée en ce que** la substance de conjugaison contient :
comme composant à activité immunologique :
a) une molécule réceptrice voire son fragment à affinité pour un épitope de la protéine structurale virale exprimée sur la membrane plasmique de cellules infectées, ou
b) un fragment modifié, par mutagenèse, de la molécule cellulaire réceptrice à affinité pour un épitope de la protéine structurale virale exprimée sur la membrane plasmique de cellules infectées,
et comme composant radioactif,
c) un radiotraceur alpha ou un radiotraceur bêta.

2. Mise en oeuvre d'une substance de conjugaison radio-immunologique (SCRI) en vue de l'élaboration d'un agent thérapeutique pour le traitement d'infections virales et de tumeurs induites par ces dernières,
**caractérisée en ce que** la substance de conjugaison contient :
comme composant à activité immunologique :
a) un anticorps monoclonal voire son fragment liant l'antigène contre un antigène viral ou induit par virus exprimé sur la membrane plasmique de cellules infectées par virus,
et comme composant radioactif,
b) un radiotraceur alpha ou un radiotraceur bêta,
et **caractérisée en outre en ce que** :
c) l'agent thérapeutique destiné une infection à VIH est préparé en vue d'une administration à la suite de ou pendant une thérapie antirétrovirale comme la triple thérapie standard, et/ou
d) l'agent thérapeutique destiné à une infection à VHB, VHC ou VHD est préparé en vue d'une administration à la suite de ou pendant une monothérapie par IFN-α ou une thérapie combinée à la ribavirine,
avec la restriction que la SCRI n'est pas un agent thérapeutique pour le traitement d'infections à VIH et de tumeurs induites par ces dernières, lorsque l'anticorps monoclonal voire son fragment contient, à titre de composant radioactif, un radiotraceur bêta.

3. Mise en oeuvre selon la revendication 1 ou 2, **caractérisée en ce que** l'infection virale est une infection à VIH, VHB, VHC, VHD, HTLV, CMV, EBV ou VHH8.

4. Mise en oeuvre selon la revendication 3, **caractérisée en ce que** l'infection est une infection à VIH, en particulier une infection à VIH-1, VIH-2, VIH-3, une infection à VHB, une infection à VHC, ou une infection à HTLV, en particulier une infection à HTLV-1 ou HTLV-2.

5. Mise en oeuvre selon l'une des revendications 1, 3 ou 4, **caractérisée en ce que** la substance de conjugaison radio-immunologique contient, à titre de composant à activité immunologique, le récepteur CD4 voire un fragment de celui-ci à affinité pour un épitope de gp120 de VIH-1 et/ou VIH-2.

6. Mise en oeuvre selon l'une des revendications 1, 3 ou 4, **caractérisée en ce que** la substance de conjugaison radio-immunologique contient, à titre de composant à activité immunologique, le récepteur LDL ou un autre récepteur à affinité pour un épitope sur la glycoprotéine de surface E1 voire E2 du VHC voire un fragment de ces récepteurs.

7. Mise en oeuvre selon l'une des revendications 1, 3 ou 4, **caractérisée en ce que** la substance de conjugaison radio-immunologique contient, à titre de composant à activité immunologique, le récepteur ASGPR ou un autre récepteur à affinité pour des glycoprotéines de surface du VHB voire un fragment de ces récepteurs.

8. Mise en oeuvre selon l'une des revendications qui précèdent, **caractérisée en ce que** la substance de conjugaison radio-immunologique est préparée en vue d'une administration intraveineuse, en une ou plusieurs fois, d'une dose corporelle totale de 25 à 300 mCi, avec couverture du patient sous irradiation pendant un à plusieurs jours à l'abri de l'environnement

9. Mise en oeuvre selon l'une des revendications qui précèdent, **caractérisée en ce que** le radiotraceur α ou β a une durée de demi-vie de 10 jours et moins.

10. Mise en oeuvre selon la revendication 9, **caractérisée en ce que** le radiotraceur α est ou contient du bismuth 212 et/ou du bismuth 213 et/ou de l'astate 211 et/ou du radium 223 et/ou de l'actinium 225, et le radiotraceur β, de l^{,131}I et/ou du ⁸⁹Sr, et/ou du ¹⁷⁷Lu, et/ou du ¹⁸⁶Lu et/ou du ¹⁸⁶Re, et/ou du ¹⁸⁸Re, et/ou du ¹⁰⁵Rh, et/ou du ⁴⁷Sc, et/ou du ¹⁵³Sm et/ou du ¹⁴⁹Tb.

11. Mise en oeuvre selon l'une des revendications qui précèdent, **caractérisée en ce que** :
a) l'agent thérapeutique destiné à une infection à VIH est préparé en vue de l'administration à la suite de ou pendant une thérapie antirétrovirale, comme la triple thérapie standard, et/ou
b) l'agent thérapeutique destiné à une infection à VHB, VHC ou VHD est préparé en vue d'une administration à la suite de ou pendant une monothérapie par IFN-α ou une thérapie combinée à la ribavirine, et/ou
c) l'agent thérapeutique est préparé en vue d'une administration avant, pendant ou après une intervention opératoire, en particulier transplantation d'un foie à transformation cirrhotique consécutive à une hépatite virale, ou résection d'un carcinome hépatocellulaire induit par hépatite virale, et/ou
d) l'agent thérapeutique est préparé en vue d'une administration sous la protection d'une transplantation de cellules de souche, et/ou
e) l'agent thérapeutique est préparé sans composant radioactif en vue d'une administration après, avant ou simultanément à une administration d'un composant immunologique tel que défini à la revendication 1 ou 2.

12. Kit comprenant un ou plusieurs composants immunologiques tels que définis dans les revendications 1 ou 2, et une colonne chromatographique.

13. Kit selon la revendication 12, comprenant en outre un solvant pour le(s) composant(s) immunologique(s) ainsi qu'éventuellement un agent de couplage, comme la chloramine T, pour l'élaboration de la substance de conjugaison (SCRI).
